(19) Europäisches Patentamt
European Patent Office
Office européen des brevets

(11) Veröffentlichungsnummer: **0 011 246**
**A1**

# EUROPÄISCHE PATENTANMELDUNG

(21) Anmeldenummer: 79104394.6

(22) Anmeldetag: 08.11.79

(51) Int. Cl.[3]: C 07 D 471/18
A 61 K 31/50
//(C07D471/18, 237/00, 221/00, 221/00), (C07D471/18, 237/00, 221/00, 209/00)

(30) Priorität: 10.11.78 CH 11585/78

(43) Veröffentlichungstag der Anmeldung: 28.05.80 Patentblatt 80/11

(84) Benannte Vertragsstaaten: AT BE CH DE FR GB IT LU NL SE

(71) Anmelder: Byk Gulden Lomberg Chemische Fabrik GmbH
Byk-Gulden-Strasse 2
D-7750 Konstanz(DE)

(72) Erfinder: Amschler, Hermann, Dr.
Hohenhewenstrasse 19
D-7760 Radolfzell(DE)

(72) Erfinder: Ulrich, Wolf-Rüdiger, Dr.
Brandesstrasse 14
D-7750 Konstanz(DE)

(72) Erfinder: Dittmann, Ernst-Christian, Dr. Dr.
Ringstrasse 123a
D-7750 Konstanz(DE)

(54) **Substituierte Pyridazine, ihre Verwendung und Herstellung und sie enthaltende Arzneimittel.**

(57) Substituierte Pyridazine der allgemeinen Formel H

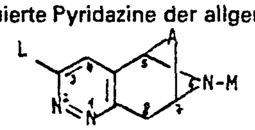 (H),

worin A eine Niederalkylengruppe, L eine Fluchtgruppe oder die Gruppe -NH-$R^1$, M ein Wasserstoffatom oder die Gruppe -C(=X)$R^2$, $R^1$ eine gegebenenfalls substituierte oder derivatisierte Aminogruppe, $R^2$ eine Alkylgruppe, eine Alkoxygruppe, eine Alkylmercaptogruppe, eine gegebenenfalls substituierte Arylgruppe, eine Phenalkoxygruppe oder eine gegebenenfalls substituierte Aminogruppe und X ein Sauerstoffatom oder ein Schwefelatom darstellt sowie ihre Säureadditionssalze mit anorganischen oder organischen Säuren sind neue Verbindungen. Sie werden in der pharmazeutischen Industrie als Zwischenprodukte und zur Herstellung blutdrucksenkender Medikamente verwendet. Die Erfindung betrifft Verbindungen der Formel H, Verfahren zu ihrer Herstellung, ihre Verwendung sowie Arzneimittel, welche diese Verbindungen enthalten.

EP 0 011 246 A1

Die Erfindung betrifft substituierte Pyridazine, ihre Verwendung und Herstellung und sie enthaltende Arzneimittel.

Die erfindungsgemäßen Verbindungen werden in der pharmazeutischen Industrie als Zwischenprodukte und zur Herstellung von Medikamenten verwendet.

1-Hydrazinophthalazin (Hydralazin), seine blutdrucksenkende Wirksamkeit und die Nebenwirkungen sind bekannt (Ehrhard/Ruschig: Arzneimittel, 2.Auflage, Band 2, Seite 278/79, Verlag Chemie, Weinheim/Bergstraße, 1972). In den deutschen Offenlegungsschriften DE-OS 22 21 808 und DE-OS 24 36 417 werden Hydralazin-Abkömmlinge beschrieben, denen ebenfalls antihypertensive Eigenschaften zugesprochen werden.

Es wurde nun eine neue Klasse von substituierten Pyridazinen synthetisiert, die weder in den erwähnten Publikationen genannt noch durch sie nahegelegt wird. Ferner wurde gefunden, daß diese substituierten Pyridazine interessante, besonders vorteilhafte pharmakologische Eigenschaften aufweisen.

Gegenstand der Erfindung sind substituierte Pyridazine der allgemeinen Formel H

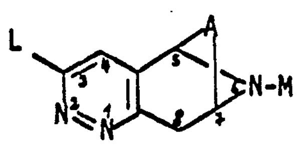 (H),

worin

A eine Niederalkylengruppe,
L eine Fluchtgruppe Z oder die Gruppe $-NH-R^1$,
M ein Wasserstoffatom oder die Gruppe $-C(=X)R^2$,
$R^1$ eine gegebenenfalls substituierte oder derivatisierte Aminogruppe,
$R^2$ eine Alkylgruppe, eine Alkoxygruppe, eine Alkylmercaptogruppe, eine gegebenenfalls substituierte Arylgruppe, eine Phenalkoxygruppe oder eine gegebenenfalls substituierte Aminogruppe und

X ein Sauerstoffatom oder ein Schwefelatom darstellt, sowie ihre Säureadditionssalze mit anorganischen oder organischen Säuren.

Als Niederalkylengruppen kommen solche in Frage, die 1 bis 5, vorzugsweise 2 bis 3 Kohlenstoffatome aufweisen. Beispielsweise seien genannt die Methylen-, Ethylen-, Propylen- und die 2,2-Dimethylpropylengruppe. Bevorzugt sind die Ethylen- und die Propylengruppe.

Die Fluchtgruppe Z kann beispielsweise eine Alkoxygruppe, wie z.B. eine Methoxy- oder Ethoxygruppe, oder eine Mercapto- oder Alkylmercapto-, wie z.B. eine Methylmercaptogruppe, oder eine Alkylsulfinyl- oder Alkylsulfonyl-, wie z.B. eine Ethylsulfonylgruppe, oder bevorzugt ein Halogenatom, insbesondere ein Brom- oder Chloratom, sein.

Eine gegebenenfalls substituierte Aminogruppe $R^1$ kann durch $-NH-R^3$ oder $-N(R^4)R^5$ charakterisiert werden, wobei $R^3$ ein Wasserstoffatom, eine Alkanoylgruppe, eine Alkoxycarbonylgruppe oder eine (Di)alkylcarbamoylgruppe bedeutet, $R^4$ und $R^5$ gleich oder verschieden sind und eine Niederalkylgruppe bedeuten. Eine gegebenenfalls substituierte Aminogruppe $R^2$ kann durch $-N(R^6)R^7$ charakterisiert werden, worin $R^6$ und $R^7$ gleich oder verschieden sind und ein Wasserstoffatom, eine Alkylgruppe oder eine gegebenenfalls substituierte Arylgruppe bedeuten oder gemeinsam unter Einschluß des Stickstoffatoms einen gegebenenfalls substituierten heterocyclischen Rest darstellen.

Unter einer derivatisierten Aminogruppe wird eine $-N=C(R^8)R^9$-Gruppe verstanden, worin $R^8$ ein Wasserstoffatom, eine Alkylgruppe oder eine gegebenenfalls substituierte Arylgruppe und $R^9$ eine Alkylgruppe, eine Alkenylgruppe oder eine gegebenenfalls substituierte Arylgruppe bedeuten oder $R^8$ und $R^9$ gemeinsam eine Alkylengruppe mit 4 bis 11, vorzugsweise 5 bis 7 Konlenstoffatomen darstellen.

Als Alkylreste kommen geradkettige oder verzweigte Alkylreste mit 1 bis 7 Kohlenstoffatomen in Frage. Geradkettige Alkylreste sind der Methyl-, Ethyl-, Propyl-, Butyl-, Pentyl-, Hexyl- und Heptylrest, von denen die Niederalkylreste mit 1 bis 5, vor allem mit 1 und 2 Kohlenstoffatomen bevorzugt sind. Verzweigte Alkylreste mit 3 bis 7 Kohlenstoffatomen sind z.B. der Isopropyl-, Isobutyl-, sek.-Butyl- oder tert.-Butylrest, von denen die mit 3 bis 5, vor allem mit 4 Kohlenstoffatomen bevorzugt sind. Zu den Alkylresten werden auch Phenalkylreste gerechnet, z.B. der Benzylrest. Als Alkenylreste kommen solche mit 3 bis 7, vorzugsweise 3 bis 5 Kohlenstoffatomen in Betracht. Beispielsweise seien genannt der Allylrest, 2-Butenylrest und 2- Pentenylrest.

Alkoxy- bzw. Alkylmercaptogruppen enthalten neben dem Heteroatom die vorstehend angegebenen Alkylreste mit 1 bis 7 Kohlenstoffatomen, vorzugsweise Niederalkylreste mit 1 bis 5 Kohlenstoffatomen. Alkanoylreste leiten sich von Alkancarbonsäuren mit 2 bis 7, vorzugsweise von Niederalkancarbonsäuren mit 2 bis 5 Kohlenstoffatomen ab.

Stellen $R^6$ und $R^7$ gemeinsam unter Einschluß des Stickstoffatoms einen gegebenenfalls substituierten heterocyclischen Rest dar, so ist dies bevorzugt ein fünf- bis sechsgliedriger Ring, der neben dem Stickstoffatom ein Sauerstoffatom oder ein Schwefelatom oder ein gegebenenfalls durch eine Niederalkylgruppe substituiertes Stickstoffatom enthalten kann. Beispielsweise seien genannt die Pyrrolidino-, Piperidino-, Morpholino-, Thiomorpholino-, Piperazino- und 4-Methylpiperazinogruppe.

Als Arylreste, die gegebenenfalls substituiert sein können, kommen solche mit 6 bis 10 Kohlenstoffatomen in Frage, beispielsweise der Phenyl- oder Naphthylrest, insbesondere der Phenylrest. Die Arylreste können ferner in beliebiger Stellung, beispielsweise mit 2, vorzugsweise mit 1 Substituenten, substituiert sein, wobei die energetisch begünstigten Stellen bevorzugt sind. Als Substituenten seien u.a. genannt Halogenatome, beispielsweise Fluor und Brom, vorzugs-

BAD ORIGINAL

weise Chlor, Alkyl-, Alkoxy- oder Alkylmercaptogruppen mit jeweils 1 bis 4 Kohlenstoffatomen, die Trifluormethylgruppe oder die Nitrogruppe. Beispiele für substituierte Arylgruppen sind die o-Chlorphenyl-, m-Chlorphenyl-, p-Chlorphenyl-, m-Bromphenyl-, p-Bromphenyl-, p-Fluorphenyl-, m-Tolyl-, p-Tolyl-, 3,4-Dichlorphenyl-, 3-Chlor-p-tolyl-, α,α,α-Trifluor-m-tolyl-, p-Nitrophenyl-, m-Nitrophenyl-, p-Methoxyphenyl-, p-Ethoxyphenyl-, 3,4-Dimethoxyphenyl-, Cumenyl-, p-Butylmercaptophenyl-, 4-Chlor-1-naphthylgruppe, von denen die durch ein Halogenatom substituierten Phenylgruppen bevorzugt sind.

Als Salze kommen alle Säureadditionssalze in Betracht. Besonders erwähnt seien die pharmakologisch verträglichen Salze der in der Galenik üblicherweise verwendeten anorganischen und organischen Säuren. Pharmakologisch unverträgliche Salze werden durch dem Fachmann bekannte Verfahren in pharmakologisch verträgliche Salze übergeführt. Als solche eignen sich beispielsweise wasserlösliche und wasserunlösliche Säureadditionssalze, wie das Hydrochlorid, Hydrobromid, Hydrojodid, Phosphat, Nitrat, Sulfat, Acetat, Citrat, Gluconat, Benzoat, Hibenzat (2-(4-Hydroxybenzoyl)-benzoat), Fendizoat (o-[(2'-Hydroxy-4-biphenylyl)-carbonyl]-benzoat), Propionat, Butyrat, Sulfosalicylat, Maleat, Laurat, Malat, Fumarat, Succinat, Oxalat, Tartrat, Amsonat (4,4'-Diamino-stilben-2,2'-disulfonat), Embonat (1,1'-Methylen-bis-2-hydroxy-3-naphthoat), Metembonat, Stearat, Tosilat (p-Toluolsulfonat), 2-Hydroxy-3-naphthoat, 3-Hydroxy-2-naphthoat, Mesilat (Methansulfonat), ferner Salze mit Bumetanid (3-(Butylamino)-4-phenoxy-5-sulfamoyl-benzoesäure), Furosemid (4-Chlor-N-furfuryl-5-sulfamoylanthranilsäure), Azosemid (5-(4-Chlor-5-sulfamoyl-2-thenylamino)-phenyltetrazol), Galosemid (N-{[4-α,α,α-Trifluor-m-toluidino)-3-pyridil]-sulfonyl}-propionamid, Besunid (4-Benzyl-3-(butylamino)-5-sulfamoyl-benzoesäure), Piretanid (4-Phenoxy-3-(1-pyrrolidinyl)-5-sulfamoyl-benzoesäure), Etacrynsäure ([2,3-Dichlor-4-(2-methylenbutyryl)-phenoxy]-essigsäure), Tienilinsäure ([2,3-Dichlor-4-(2-thenoyl)-phenoxy]-essigsäure), 4-Chlor-3-sulfamoyl-benzoesäure.

Eine Ausgestaltung der Erfindung sind Pyridazine der allgemeinen Formel I

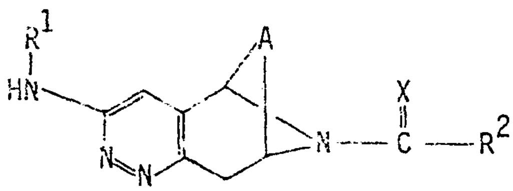

(I),

worin

A eine Niederalkylengruppe,

$R^1$ eine gegebenenfalls substituierte oder derivatisierte Aminogruppe,

$R^2$ eine Alkylgruppe, eine Alkoxygruppe, eine Alkylmercaptogruppe, eine gegebenenfalls substituierte Arylgruppe, eine Phenalkoxygruppe oder eine gegebenenfalls substituierte Aminogruppe darstellt und

X ein Sauerstoffatom oder ein Schwefelatom bedeutet,

sowie ihre Säureadditionssalze mit anorganischen und organischen Säuren.

Eine Ausgestaltung der Verbindungen der allgemeinen Formel I sind Verbindungen der allgemeinen Formel I*

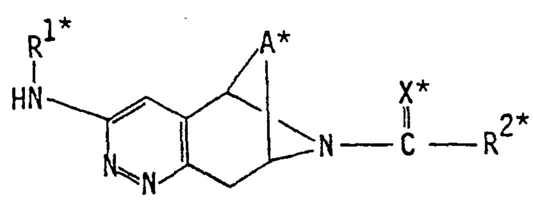

(I*),

worin

A* einen Alkylenrest mit 2 bis 3 Kohlenstoffatomen,

$R^{1*}$ eine $-NH-R^{3*}$-Gruppe oder eine $-N{=}C(R^{8*})R^{9*}$-Gruppe,

$R^{2*}$ eine Niederalkylgruppe, eine Niederalkoxygruppe, eine gegebenenfalls substituierte Phenylgruppe oder eine $-N(R^{6*})R^{7*}$-Gruppe,

BAD ORIGINAL

$R^{3*}$ ein Wasserstoffatom, eine Niederalkanoylgruppe oder eine Niederalkoxycarbonylgruppe,

$R^{6*}$ ein Wasserstoffatom oder eine Niederalkylgruppe,

$R^{7*}$ eine Niederalkylgruppe oder eine gegebenenfalls substituierte Phenylgruppe bedeuten oder

$R^{6*}$ und $R^{7*}$ gemeinsam unter Einschluß des Stickstoffatoms einen Piperidino-, Morpholino-, Piperazino- oder 4-Niederalkylpiperazinorest darstellen,

$R^{8*}$ eine Niederalkylgruppe oder eine gegebenenfalls substituierte Phenylgruppe,

$R^{9*}$ eine Niederalkylgruppe oder eine gegebenenfalls substituierte Phenylgruppe und

X* ein Sauerstoffatom bedeuten,

und ihre Säureadditionssalze.

Eine weitere Ausgestaltung der Verbindungen der allgemeinen Formel I sind Verbindungen der allgemeinen Formel I**

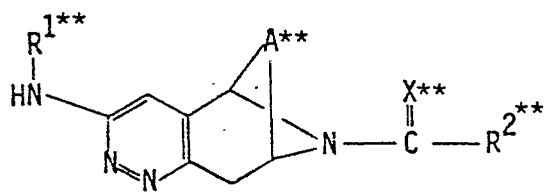

(I**),

worin

A** einen Alkylenrest mit 2 bis 3 Kohlenstoffatomen,

$R^{1**}$ eine $-NH-R^{3**}$-Gruppe oder eine $-N=C(R^{8**})R^{9**}$-Gruppe,

$R^{2**}$ eine Niederalkyl- oder Niederalkoxygruppe, eine Phenylgruppe oder eine $-N(R^{6**})R^{7**}$-Gruppe,

$R^{3**}$ ein Wasserstoffatom oder eine Niederalkoxycarbonylgruppe,

$R^{6**}$ ein Wasserstoffatom,

$R^{7**}$ eine Niederalkylgruppe oder eine Phenylgruppe,

$R^{8**}$ eine Niederalkylgruppe,

$R^{9**}$ eine Niederalkylgruppe oder eine gegebenenfalls halogenmonosubstituierte Phenylgruppe und

X** ein Sauerstoffatom bedeuten,

und ihre Säureadditionssalze.

BAD ORIGINAL

Bevorzugte Vertreter der Ausgestaltung I** sind solche, in denen A**, $R^{1**}$ und $R^{6**}$ die oben angegebenen Bedeutungen haben, $R^{2**}$ eine tert.-Butyl-, eine Ethoxy-, eine Phenyl- oder eine -N($R^{6**}$)$R^{7**}$-Gruppe, $R^{3**}$ ein Wasserstoffatom oder eine Ethoxycarbonylgruppe, $R^{7**}$ eine Methylgruppe, $R^{8**}$ eine Methylgruppe, $R^{9**}$ eine Methyl-, Ethyl- oder 4-Chlorphenylgruppe bedeutet und X** ein Sauerstoffatom darstellt, sowie ihre Säureadditionssalze mit anorganischen oder organischen Säuren.

Besonders bevorzugte Vertreter der Ausgestaltung I** sind solche, in denen A** und $R^{1**}$ die oben angegebenen Bedeutungen haben, $R^{2**}$ eine Ethoxy- oder eine Phenylgruppe, $R^{3**}$ ein Wasserstoffatom, $R^{8**}$ eine Methylgruppe, $R^{9**}$ eine Methylgruppe und X** ein Sauerstoffatom darstellt, sowie ihre pharmakologisch verträglichen Säureadditionssalze mit anorganischen oder organischen Säuren.

Eine weitere Ausgestaltung der Erfindung sind Verbindungen der allgemeinen Formel II

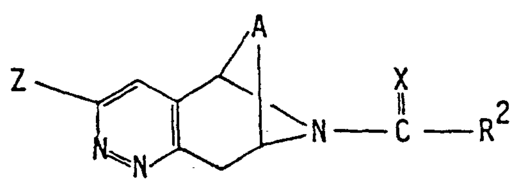

(II),

worin A, Z, X und $R^2$ die oben angegebene Bedeutung haben.

Eine Ausgestaltung der Verbindungen der allgemeinen Formel II sind Verbindungen der allgemeinen Formel II*,

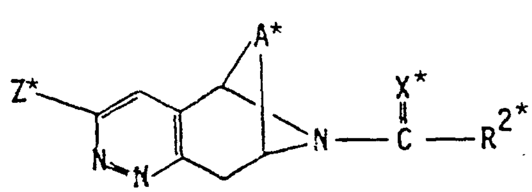

(II*),

worin Z* ein Halogenatom bedeutet und A*, X* und $R^{2*}$ die oben angegebene Bedeutung haben.

BAD ORIGINAL

Eine weitere Ausgestaltung der Verbindungen der allgemeinen Formel II sind Verbindungen der allgemeinen Formel II**,

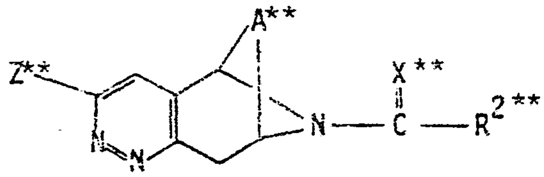 (II**),

worin Z** ein Brom- oder Chloratom bedeutet und A**, X** und $R^{2**}$ die oben angegebene Bedeutung haben.

Eine weitere Ausgestaltung der Erfindung sind Verbindungen der allgemeinen Formel II'

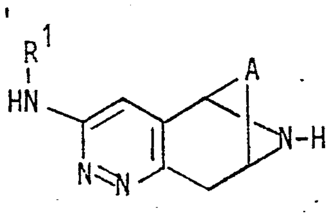 (II'),

worin A und $R^1$ die oben angegebene Bedeutung haben, und ihre Säureadditionssalze.

Eine Ausgestaltung der Verbindungen der allgemeinen Formel II' sind Verbindungen der allgemeinen Formel II'*

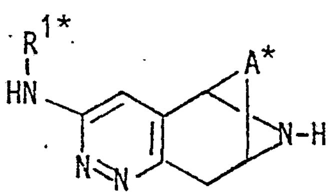 (II'*),

worin A* und $R^{1*}$ die oben angegebene Bedeutung haben, und ihre Säureadditionssalze.

Eine weitere Ausgestaltung der Verbindungen der allgemeinen Formel II' sind Verbindungen der allgemeinen Formel II'**

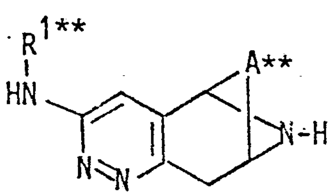 (II'**),

BAD ORIGINAL

worin A** und $R^{1**}$ die oben angegebene Bedeutung haben, und ihre Säureadditionssalze.

Eine weitere Ausgestaltung der Erfindung sind Verbindungen der allgemeinen Formel VI

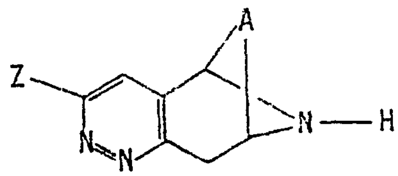

(VI),

worin Z und A die oben angegebenen Bedeutungen haben, und ihre Säureadditionssalze.

Eine Ausgestaltung der Verbindungen der allgemeinen Formel VI sind Verbindungen der allgemeinen Formel VI*

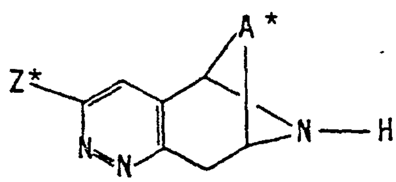

(VI*),

worin Z* und A* die oben angegebenen Bedeutungen haben, und ihre Säureadditionssalze.

Eine weitere Ausgestaltung der Verbindungen der allgemeinen Formel VI sind Verbindungen der allgemeinen Formel VI**

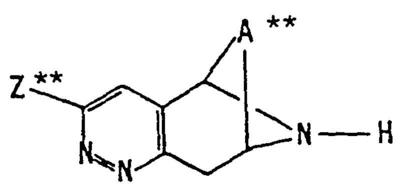

(VI**),

worin Z** und A** die oben angegebene Bedeutung haben, und ihre Säureadditionssalze.

Bei den erfindungsgemäßen Verbindungen handelt es sich um chirale Moleküle. Die Erfindung schließt daher sowohl die Enantiomeren als auch deren Gemische und Racemate ein.

BAD ORIGINAL

Als Vertreter erfindungsgemäßer Verbindungen seien beispielsweise genannt:

3-Hydrazino-5,7-propano-5,6,7,8-tetrahydro-6-pyrido-[4,3-c]-pyridazincarbonsäurebenzylester

3-Hydrazino-5,7-propano-5,6,7,8-tetrayhdro-6-pyrido-[4,3-c]-pyridazincarbonsäure-tert.-butylester

3-Hydrazino-5,7-propano-5,6,7,8-tetrahydro-6-pyrido-[4,3-c]-pyridazincarbonsäurepentylester

3-(1,3-Dimethyl-2-butenyliden-hydrazino)-5,7-propano-5,6,7,8-tetrahydro-6-pyrido-[4,3-c]-pyridazincarbonsäure-tert.-butylester

3-Cyclopentyliden-hydrazino-5,7-propano-5,6,7,8-tetrahydro-6-pyrido-[4,3-c]-pyridazincarbonsäurebenzylester

3-(2-Ethoxycarbonyl-hydrazino)-5,7-propano-5,6,7,8-tetrahydro-6-pyrido-[4,3-c]-pyridazincarbonsäure-N-methylamid

3-Isopropyliden-hydrazino-5,7-propano-5,6,7,8-tetrahydro-6-pyrido-[4,3-c]-pyridazincarbonsäure-N,N-dimethylamid

3-(2-Acetyl-hydrazino)-5,7-propano-5,6,7,8-tetrahydro-6-pyrido-[4,3-c]-pyridazincarbonsäureethylester

3-(1-Phenylethyliden-hydrazino)-5,7-propano-5,6,7,8-tetrahydro-6-pyrido-[4,3-c]-pyridazincarbonsäure-N-methylamid

3-Hydrazino-6-pivaloyl-5,7-propano-5,6,7,8-tetrahydro-pyrido-[4,3-c]-pyridazin

6-Butyryl-3-hydrazino-5,7-propano-5,6,7,8-tetrahydro-pyrido-[4,3-c]-pyridazin

3-Butyliden-hydrazino-6-propionyl-5,7-propano-5,6,7,8-tetrahydro-pyrido-[4,3-c]-pyridazin

3-(1,3-Dimethyl-2-butenyliden-hydrazino)-6-(3,4-dichlorbenzoyl)-5,7-propano-5,6,7,8-tetrahydro-pyrido-[4,3-c]-pyridazin

3-Hydrazino-6-p-toluolyl-5,7-propano-5,6,7,8-tetrahydro-pyrido[4,3-c]-pyridazin

6-(4-Chlorbenzoyl)-3-(2-methoxycarbonyl-hydrazino)-5,7-propano-5,6,7,8-tetrahydro-pyrido-[4,3-c]-pyridazin

3-Hydrazino-6-pivaloyl-5,7-ethano-5,6,7,8-tetrahydro-pyrido-[4,3-c]-pyridazin

6-(2-Chlorbenzoyl)-3-hydrazino-5,7-ethano-5,6,7,8-tetrahydro-pyrido-[4,3-c]-pyridazin

BAD ORIGINAL

3-Hydrazino-5,7-ethano-5,6,7,8-tetrahydro-6-pyrido-[4,3-c]-pyridazin-carbonsäurebenzylester
3-Hydrazino-5,7-ethano-5,6,7,8-tetrahydro-6-pyrido-[4,3-c]-pyridazin-carbonsäure-tert.-butylester
3-Cyclohexyliden-hydrazino-5,7-ethano-5,6,7,8-tetrahydro-6-pyrido-[4,3-c]-pyridazincarbonsäureethylester
3-Brom-5,7-propano-5,6,7,8-tetrahydro-6-pyrido-[4,3-c]-pyridazincarbonsäureethylester
3-Chlor-5,7-propano-5,6,7,8-tetrahydro-6-pyrido-[4,3-c]-pyridazincarbonsäure-N,N-dimethylamid
3-Chlor-6-(4-chlorbenzoyl)-5,7-propano-5,6,7,8-tetrahydro-pyrido-[4,3-c]-pyridazin
3-Hydrazino-5,7-ethano-5,6,7,8-tetrahydro-pyrido-[4,3-c]-pyridazin
3-(2-Methoxycarbonyl-hydrazino)-5,7-propano-5,6,7,8-tetrahydro-pyrido-[4,3-c]-pyridazin
3-(1-p-Chlorphenylethyliden-hydrazino)-5,7-propano-5,6,7,8-tetrahydro-pyrido-[4,3-c]-pyridazin
und ihre Säureadditionssalze.

Bevorzugte Vertreter sind:

6-Benzoyl-3-isopropyliden-hydrazino-5,7-propano-5,6,7,8-tetrahydro-pyrido-[4,3-c]-pyridazin
3-Isopropyliden-hydrazino-5,7-ethano-5,6,7,8-tetrahydro-6-pyrido-[4,3-c]-pyridazincarbonsäureethylester
3-Chlor-5,7-propano-5,6,7,8-tetrahydro-6-pyrido-[4,3-c]-pyridazin-carbonsäureethylester
3-Chlor-5,7-ethano-5,6,7,8-tetrahydro-6-pyrido-[4,3-c]-pyridazin-carbonsäureethylester
6-Benzoyl-3-chlor-5,7-propano-5,6,7,8-tetrahydro-pyrido-[4,3-c]-pyridazin
3-Chlor-6-pivaloyl-5,7-propano-5,6,7,8-tetrahydro-pyrido-[4,3-c]-pyridazin
3-Chlor-5,7-propano-5,6,7,8-tetrahydro-6-pyrido-[4,3-c]-pyridazin-carbonsäure-N-methylamid
3-Isopropylidenhydrazino-5,7-propano-5,6,7,8-tetrahydro-pyrido-[4,3-c]-pyridazin
3-Chlor-5,7-propano-5,6,7,8-tetrahydro-pyrido-[4,3-c]-pyridazin, insbesondere

3-Hydrazino-5,7-propano-5,6,7,8-tetrahydro-6-pyrido-[4,3-c]-pyridazin-carbonsäureethylester
und ihre Säureadditionssalze.

Die erfindungsgemäßen Verbindungen besitzen wertvolle Eigenschaften. Die substituierten Pyridazine der allgemeinen Formel I, I*, I**, ihre bevorzugten Vertreter und ihre Säureadditionssalze sind erfindungsgemäße pharmakologisch wirksame Verbindungen; insbesondere senken sie den Blutdruck, wie aus Untersuchungen an wachen, genetisch hypertonen Ratten hervorgeht. Die erfindungsgemäßen pharmakologisch wirksamen Verbindungen, z.B. 3-Hydrazino-5,7-propano-5,6,7,8-tetrahydro-6-pyrido-[4,3-c]-pyridazin-carbonsäureethylester, weisen dabei eine erheblich höhere antihypertensive Wirksamkeit und längere Wirkungsdauer auf als Hydralazin bzw. die Verbindungen der DE-OS 22 21 808 und DE-OS 24 36 417. Darüberhinaus sind sie gegenüber dem Stand der Technik durch eine bessere Verträglichkeit gekennzeichnet, wie Bestimmungen der $LD_{50}$ nach einmaliger oraler Gabe an der Maus im Vergleich mit Hydralazin ergaben. Im Hinblick auf die Eigenschaften, insbesondere die erhöhte Wirksamkeit und längere Wirkungsdauer, stellen die erfindungsgemäßen pharmakologisch wirksamen Verbindungen einen therapeutischen Fortschritt und eine erwünschte Bereicherung des Standes der Technik dar. Die erfindungsgemäßen substituierten Pyridazine der allgemeinen Formeln II, II*, II**, II', II'*, II'**, VI, VI* und VI** sind wertvolle Zwischenprodukte für die Herstellung der pharmakologisch wirksamen erfindungsgemäßen Verbindungen.

Die ausgezeichnete Wirksamkeit der erfindungsgemäßen pharmakologisch wirksamen Verbindungen I, I*, I**, ihrer bevorzugten Vertreter und ihrer Säureadditionssalze gestattet ihren Einsatz in der Humanmedizin, wobei als Indikationen besonders primäre und sekundäre Hypertonien aller Schweregrade in Betracht kommen.

Ein weiterer Gegenstand der Erfindung ist daher ein Verfahren zur Behandlung von Säugetieren, die an einer der obengenannten Krankheiten erkrankt sind. Das Verfahren ist dadurch gekennzeichnet, daß man dem erkrankten Säugetier eine therapeutisch wirksame und pharmakologisch verträgliche Menge einer oder mehrerer Verbindungen der allgemeinen Formeln I, I*, I**, deren bevorzugte Vertreter und/oder deren Salze verabreicht. Gegenstand der Erfindung ist außerdem die Verwendung der erfindungsgemäßen pharmakologisch wirksamen Verbindungen bei der Bekämpfung der oben angegebenen Krankheiten. Ebenso umfaßt die Erfindung die Verwendung dieser Verbindungen zur Herstellung von Arzneimitteln, die zur Bekämpfung der angeführten Krankheiten eingesetzt werden.

Ein weiterer Gegenstand der Erfindung sind daher Arzneimittel, die ein oder mehrere substituierte Pyridazine der allgemeinen Formel I

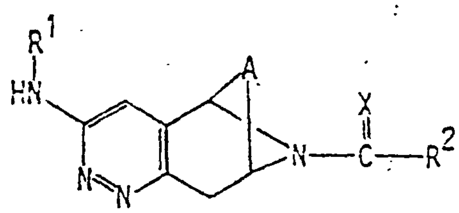

(I),

worin

A eine Niederalkylengruppe,

$R^1$ eine gegebenenfalls substituierte oder derivatisierte Aminogruppe,

$R^2$ eine Alkylgruppe, eine Alkoxygruppe, eine Alkylmercaptogruppe, eine gegebenenfalls substituierte Arylgruppe, eine Phenalkoxygruppe oder eine gegebenenfalls substituierte Aminogruppe und

X ein Sauerstoffatom oder ein Schwefelatom darstellt,

und/oder ihre pharmakologisch verträglichen Säureadditionssalze mit anorganischen oder organischen Säuren enthalten.

Ausgestaltungen der Arzneimittel sind solche, die substituierte Pyridazine der Formel I*, I** oder ihre bevorzugten Vertreter und/oder ihre pharmakologisch verträglichen Säureadditionssalze mit anorganischen oder organischen Säuren enthalten.

Die Arzneimittel werden nach an sich bekannten Verfahren hergestellt, wobei die neuen Verbindungen als solche oder gegebenenfalls in Kombination mit geeigneten pharmazeutischen Trägerstoffen eingesetzt werden. Enthalten die neuen pharmazeutischen Zubereitungen neben den Wirkstoffen pharmazeutische Trägerstoffe, beträgt der Wirkstoffgehalt dieser Mischungen 0,1 bis 99,5, vorzugsweise 0,5 bis 95 Gewichtsprozent der Gesamtmischung.

In Übereinstimmung mit der Erfindung werden die Wirkstoffe in jeder geeigneten Formulierung angewandt unter der Voraussetzung, daß die Ausbildung bzw. Aufrechterhaltung von ausreichenden Blutspiegeln gewährleistet ist. Das kann beispielsweise durch orale oder parenterale Gabe in geeigneten Dosen erreicht werden. Vorteilhafterweise liegt die pharmazeutische Zubereitung des Wirkstoffes in Form von Einheitsdosen vor, die auf die gewünschte Verabreichung abgestimmt sind. Eine Einheitsdosis kann zum Beispiel eine Tablette, ein Drageé, eine Kapsel, ein Suppositorium oder eine gemessene Volumenmenge eines Pulvers, eines Granulates, einer Lösung, einer Emulsion oder einer Suspension sein.

Unter "Einheitsdosis" im Sinne der vorliegenden Erfindung wird eine physikalisch bestimmte Einheit, die eine individuelle Menge des aktiven Bestandteils in Kombination mit einem pharmazeutischen Trägerstoff enthält, verstanden, deren Wirkstoffgehalt einem Bruchteil oder Vielfachen einer therapeutischen Einzeldosis entspricht. Eine Einzeldosis enthält vorzugsweise die Menge Wirkstoff, die bei einer Applikation verabreicht wird und die gewöhnlich einer ganzen, einer halben, einer drittel oder einer viertel Tagesdosis entspricht. Wenn für eine einzelne therapeutische Verabreichung nur ein Bruchteil, wie die Hälfte oder ein Viertel, der Einheitsdosis benötigt wird, ist die Einheitsdosis vorteilhafterweise teilbar, z.B. in Form einer Tablette mit Bruchkerbe.

Die pharmazeutischen Zubereitungen gemäß der Erfindung können, wenn sie in Einheitsdosen vorliegen und für die Applikation z.B. am Menschen bestimmt sind, etwa 0,1 bis 500 mg, vorteilhafterweise 0,5 bis 100 mg und insbesondere 1 bis 30 mg Wirkstoff enthalten.

Im allgemeinen hat es sich in der Humanmedizin als vorteilhaft erwiesen, den oder die Wirkstoffe bei oraler Gabe in einer Tagesdosis von etwa 0,001 bis etwa 5, vorzugsweise 0,01 bis 2, insbesondere 0,05 bis 1 mg/kg Körpergewicht, gegebenenfalls in Form mehrerer, vorzugsweise 1 bis 3 Einzelgaben zur Erzielung der gewünschten Ergebnisse zu verabreichen. Eine Einzelgabe enthält den oder die Wirkstoffe in Mengen von etwa 0,001 bis etwa 2,5, vorzugsweise 0,01 bis 1,5, insbesondere 0,05 bis 0,5 mg/kg Körpergewicht. Bei einer parenteralen, z.B. intravenösen Behandlung können ähnliche Dosierungen zur Anwendung kommen.

Die therapeutische Verabreichung der pharmazeutischen Zubereitung kann 1 bis 4 mal am Tage zu festgelegten oder variierenden Zeitpunkten erfolgen, z.B. jeweils nach den Mahlzeiten und/oder am Abend. Es kann jedoch erforderlich sein, von den genannten Dosierungen abzuweichen, und zwar in Abhängigkeit von der Art, dem Körpergewicht und dem Alter des zu behandelnden Individuums, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Bei einschleichender Dosierung wird zu Beginn der Behandlung eine geringere Dosis verabreicht, dann langsam auf eine höhere Dosis übergegangen. Nach Erreichen der gewünschten Blutdrucksenkung wird wieder auf eine niedrigere Dosis zurückgegangen.

Die Festlegung der jeweils erforderlichen optimalen Dosierung und Applikationsart der Wirkstoffe kann durch jeden Fachmann aufgrund seines Fachwissens leicht erfolgen.

Die pharmazeutischen Zubereitungen bestehen in der Regel aus den erfindungsgemäßen Wirkstoffen und nichttoxischen, pharmazeutisch verträglichen Arzneimittelträgern, die als Zumischung oder Verdünnungsmittel in fester, halbfester oder flüssiger Form oder als Umhüllungsmittel, beispielsweise in Form einer Kapsel, eines Tablettenüberzugs, eines Beutels oder eines anderen Behältnisses, für den therapeutisch aktiven Bestandteil in Anwendung kommen. Ein Trägerstoff kann z.B. als Vermittler für die Arzneimittelaufnahme durch den Körper, als Formulierungshilfsmittel, als Süßungsmittel, als Geschmackskorrigenz, als Farbstoff oder als Konservierungsmittel dienen.

Zur oralen Anwendung können z.B. Tabletten, Drageés, harte und weiche Kapseln, z.B. aus Gelatine, dispergierbare Pulver, Granulate, wäßrige und ölige Suspensionen, Emulsionen, Lösungen oder Sirupe kommen.

Tabletten können inerte Verdünnungsmittel, z.B. Calciumcarbonat, Calciumphosphat, Natriumphosphat oder Lactose; Granulierungs- und Verteilungsmittel, z.B. Maisstärke oder Alginate; Bindemittel, z.B. Stärke, Gelatine oder Akaziengummi; und Gleitmittel, z.B. Aluminium- oder Magnesiumstearat, Talkum oder Silikonöl, enthalten. Sie können zusätzlich mit einem Überzug versehen sein, der auch so beschaffen sein kann, daß er eine verzögerte Auflösung und Resorption des Arzneimittels im Gastrointestinaltrakt bewirkt, so daß z.B. eine bessere Verträglichkeit, Prothrahierung oder eine Retardierung erreicht wird. Gelatinekapseln können den Arzneistoff vermischt mit einem festen, z.B. Calciumcarbonat oder Kaolin, oder einem öligen, z.B. Oliven-, Erdnuß- oder Paraffinöl, Verdünnungsmittel enthalten.

Wäßrige Suspensionen können Suspendiermittel, z.B. Natriumcarboxymethylcellulose, Methylcellulose, Hydroxypropylcellulose, Natriumalginat, Polyvinylpyrrolidon, Traganthgummi oder Akaziengummi; Dispergier- und Benetzungsmittel, z.B. Polyoxyethylenstearat, Heptadecaethylenoxycetanol, Polyoxyethylensorbitolmonooleat, Polyoxyethylensorbitanmonooleat oder Lecithin; Konservierungsmittel, z.B. Methyl- oder Propylhydroxybenzoate; Geschmacksmittel; Süßungsmittel, z.B. Saccharose, Lactose, Natriumcyclamat, Dextrose, Invertzuckersirup, enthalten.

Ölige Suspensionen können z.B. Erdnuß-, Oliven-, Sesam-, Kokos- oder Paraffinöl und Verdickungsmittel, wie z.B. Bienenwachs, Hartparaffin oder Cetylalkohol, enthalten; ferner Süßungsmittel, Geschmacksmittel und Antioxidantien.

In Wasser dispergierbare Pulver und Granulate können die Arzneistoffe in Mischung mit Dispergier-, Benetzungs- und Suspendiermitteln, z.B. den oben genannten, sowie mit Süßungsmitteln, Geschmacksmitteln und Farbstoffen enthalten.

Emulsionen können z.B. Oliven-, Erdnuß- oder Paraffinöl neben Emulgiermitteln, wie z.B. Akaziengummi, Traganthgummi, Phosphatiden, Sorbitanmonooleat, Polyoxyethylensorbitanmonooleat, und Süßungs- und Geschmacksmitteln enthalten.

Zur parenteralen Anwendung der Arzneistoffe dienen steril injizierbare wäßrige Suspensionen, isotonische Salzlösungen oder sonstige Lösungen, die Dispergier- oder Benetzungsmittel und/oder pharmakologisch verträgliche Verdünnungsmittel, z.B. Propylen- oder Butylenglykol, enthalten.

Der oder die Wirkstoffe können gegebenenfalls mit einem oder mehreren der angegebenen Träger- oder Zusatzstoffe auch in mikroverkapselter Form formuliert werden.

Sollen die erfindungsgemäßen substituierten Pyridazine und/oder ihre Säureadditionssalze zur Behandlung der Hypertonie eingesetzt werden, so können die pharmazeutischen Zubereitungen auch einen oder mehrere andere pharmakologisch aktive Bestandteile anderer Arzneimittelgrupppen, wie andere Antihypertensiva, ß-Rezeptorenblocker, Diuretika, Saluretika, Alkaloide, etc., wie Dihydralazin, Propranolol, Labetalol, Mefrusid, Clopamid, Spironolacton, Chlorthalidon, Furosemid, Polythiazid, Hydrochlorothiazid, Reserpin, Dihydroergocristin, Rescinamin, Rauwolfia-Gesamtalkaloide etc. enthalten.

Ein weiterer Gegenstand der Erfindung ist ein Verfahren zur Herstellung von substituierten Pyridazinen der allgemeinen Formel I

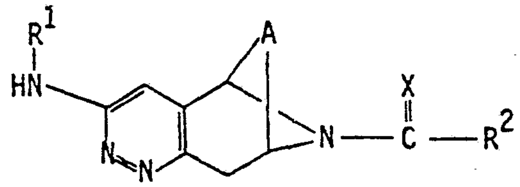

(I),

worin

A eine Niederalkylengruppe,

$R^1$ eine gegebenenfalls substituierte oder derivatisierte Aminogruppe,

$R^2$ eine Alkylgruppe, eine Alkoxygruppe, eine Alkylmercaptogruppe, eine gegebenenfalls substituierte Arylgruppe, eine Phenalkoxygruppe oder eine gegebenenfalls substituierte Aminogruppe und

X ein Sauerstoffatom oder ein Schwefelatom darstellt

und ihren Säureadditionssalzen,

das dadurch gekennzeichnet ist, daß man ein Pyridazin der allgemeinen Formel II

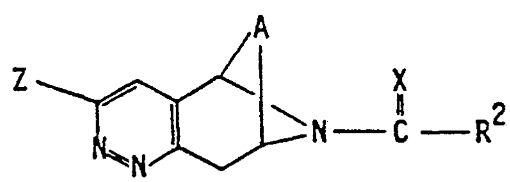

(II),

worin A, $R^2$ und X die vorstehend angegebene Bedeutung haben und Z eine Fluchtgruppe darstellt, mit einem Hydrazinderivat der allgemeinen Formel III

$$H_2N \text{———} R^1 \qquad \text{(III)},$$

worin $R^1$ die oben angegebene Bedeutung hat, umsetzt und gegebenenfalls anschließend in die Säureadditionssalze überführt oder erhaltene Reaktionsprodukte der Formel I, in denen $R^1$ eine $-NHR^3$-Gruppe und $R^3$ ein Wasserstoffatom darstellt, derivatisiert oder substituiert und/oder in die Säureadditionssalze überführt; oder daß man ein Pyridazin der allgemeinen

Formel II'

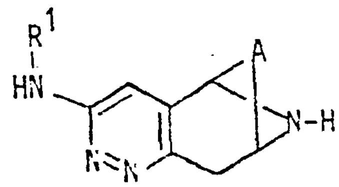

(II'),

worin $R^1$ und A die vorstehend angegebene Bedeutung haben, mit einer Verbindung der allgemeinen Formel VII

$$R^2 - C(=X)Y \qquad (VII),$$

worin $R^2$ und X die oben angegebene Bedeutung haben und Y eine Fluchtgruppe darstellt, oder (wenn $R^2$ die Bedeutung $-NHR^7$ hat und $R^7$ einen Alkyl- oder Arylrest darstellt) mit einem Heterokumulen VIII

$$R^7 - N = C = X \qquad (VIII),$$

worin $R^7$ einen Alkyl- oder Arylrest und X ein Sauerstoff- oder Schwefelatom darstellt, umsetzt und gegebenenfalls anschließend in die Säureadditionssalze überführt, oder erhaltene Reaktionsprodukte der Formel I, in denen $R^1$ eine $-NHR^3$-Gruppe und $R^3$ ein Wasserstoffatom darstellt, derivatisiert oder substituiert und/oder in die Säureadditionssalze überführt.

Zur Herstellung der Verbindungen der Ausgestaltungen I* bzw. I** und ihrer bevorzugten Vertreter werden entsprechende Ausgangsmaterialien II*, II**, II'*, II'**, III*, III**, VII*, VII**, VIII* oder VIII**, worin die Substituenten die oben angegebene Bedeutung haben, umgesetzt.

Die Reaktion der Pyridazine II mit den Hydrazinderivaten III wird nach an sich bekannten Verfahren durchgeführt. Die Fluchtgruppe Z kann beispielsweise eine Alkoxygruppe, wie z.B. eine Methoxy- oder Ethoxygruppe, oder eine Mercapto- oder Alkylmercapto-, wie z.B. eine Methylmercaptogruppe, oder eine Alkylsulfinyl- oder Alkylsulfonyl-, wie z.B. eine Ethylsulfonylgruppe, oder ein Halogenatom, wie z.B. ein

Chloratom, sein. Ist Z ein Halogenatom, vorzugsweise ein Brom- oder Chloratom, so wird die Reaktion zweckmäßigerweise in Gegenwart eines säurebindenden Mittels (Protonenacceptors) vorgenommen. Als solche sind beispielsweise geeignet Alkalimetallhydroxide, wie Natriumhydroxid, Kaliumhydroxid; oder Alkalimetallcarbonate, wie Natriumcarbonat, Kaliumcarbonat; oder tertiäre Amine, wie Pyridin, Triethylamin, Ethyldiisopropylamin. Gegebenenfalls kann auch die Verbindung III als Protonenacceptor verwendet werden. Die Umsetzung wird in diesem Fall mit einem beispielsweise fünf- bis zehnfachen Oberschuß an III bezogen auf Verbindung II durchgeführt.

Die Umsetzung wird in geeigneten, zweckmäßigerweise inerten, vorzugsweise polaren Lösungsmitteln, wie niederen Alkoholen, z.B. Methanol, Ethanol oder Isopropanol, oder offenkettigen oder cyclischen Ethern, z.B. Diethylenglycoldimethylether, Dioxan oder Tetrahydrofuran, durchgeführt. Gegebenenfalls kann auch ein Oberschuß der Verbindung III als Lösungsmittel dienen.

Die Reaktionstemperatur kann in weiten Grenzen variiert werden, z.B. 20 bis 150°C, wobei Temperaturen zwischen 80 und 120°C bevorzugt sind. Die Reaktionszeit kann zwischen 1 und 20 Stunden unter Normaldruck betragen.

Die Reaktion der Pyridazine II' mit Verbindungen der allgemeinen Formel VII wird nach an sich bekannten Verfahren durchgeführt. Die Fluchtgruppe Y kann beispielsweise eine Alkoxygruppe oder ein Halogenatom, vorzugsweise ein Chloratom sein. Die Reaktion wird zweckmäßigerweise in einem inerten, wasserfreien Lösungsmittel, z.B. einem chlorierten Kohlenwasserstoff, wie Chloroform oder 1,2-Dichlorethan und vorzugsweise unter Schutz des gegebenenfalls freien Hydrazinrestes $-NH-R^1$ durchgeführt. Ist Y ein Halogenatom, so wird die Reaktion bevorzugt in Gegenwart eines Protonenacceptors, z.B. eines Alkalimetallcarbonats, wie Natrium- oder Kaliumcarbonat, oder eines tertiären Amins, wie Triethylamin, durchgeführt. Die Reaktionstemperatur beträgt dabei -10 bis 30°C, insbesondere 0 bis 20°C. Zum Schutz des Hydrazinrestes kann dieser beispielsweise in das Hydrazon übergeführt und daraus durch schonende saure Hydrolyse wieder freigesetzt werden.

Die Reaktion der Pyridazine II' mit Heterokumulenen VIII wird vorzugsweise in inerten, wasserfreien Lösungsmitteln, z.B. chlorierten Kohlenwasserstoffen, wie Dichlormethan, oder offenkettigen oder cyclischen Ethern, wie Diethylether oder Tetrahydrofuran, oder aromatischen Kohlenwasserstoffen, wie Benzol oder Toluol, durchgeführt. Die Reaktionstemperatur beträgt zwischen -10 und 30°C, bevorzugt zwischen 0 und 20°C. Die Reaktion von II mit III ist gegenüber der von II' mit VII oder VIII bevorzugt.

Die Substitution bzw. die Derivatisierung bzw. die Oberführung in das Säureadditionssalz werden nach dem Fachmann bekannten Verfahren durchgeführt.

Die Substitution kann beispielsweise als Acylierung vorgenommen werden. Diese wird z.B. durch Umsetzung mit den entsprechenden Säureanhydriden bzw. -halogeniden in inerten Lösungsmitteln, wie z.B. chlorierten Kohlenwasserstoffen, wie Chloroform oder Methylenchlorid, oder Acetonitril, oder cyclischen oder offenkettigen Ethern, wie Dioxan, Tetrahydrofuran oder Diethylether, durchgeführt (vgl. u.a. Houben-Weyl, Bd. 8, S. 655 ff.).

Die Derivatisierung von Pyridazinen der Formel I, worin $R^1$ eine $NH_2$-Gruppe darstellt, zu Pyridazinender Formel I, worin $R^1$ die Bedeutung $-N=C(R^8)R^9$ hat und A, $R^2$ und X die oben angegebenen Bedeutungen haben, erfolgt durch Umsetzung mit Oxoverbindungen der Formel IV

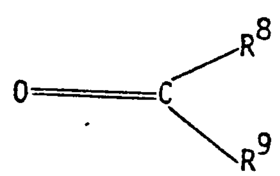 (IV),

worin $R^8$ und $R^9$ die oben angegebenen Bedeutungen haben. Die Reaktion wird gegebenenfalls unter Zusatz von einem unter den Reaktionsbedingungen inerten, polaren organischen Lösungsmittel, beispielsweise einem niederen Alkohol, wie Methanol, Ethanol oder Isopropanol oder einem offenkettigen oder cyclischen Ether, wie z.B. Diethylenglycoldimethylether, Tetrahydrofuran oder Dioxan, gegebenenfalls unter Erhitzen, indem man das Reaktionsgemisch 5 Minuten bis 10 Stunden

bei Temperaturen von ca. $0^{o}C$ bis Siedetemperatur hält und anschließend zur Trockne eindampft oder das Rohprodukt direkt oder nach weitgehendem Einengen der Lösungs auskristallisieren läßt, durchgeführt. Dieser Syntheseweg ist der oben beschriebenen einstufigen Variante vorzuziehen, die von Pyridazinen II und einem Hydrazinderivat III, worin $R^1$ die Bedeutung $-N=C(R^8)R^9$ hat, ausgeht.

Säureadditionssalze erhält man durch Auflösen der freien Base in einem geeigneten Lösungsmittel, z.B. einem chlorierten Kohlenwasserstoff, wie Methylenchlorid oder Chloroform, oder einem niedermolekularen aliphatischen Alkohol (Ethanol, Isopropanol), das die gewünschte Säure enthält, oder dem die gewünschte Säure anschließend zugegeben wird. Die Salze werden durch Filtrieren, Umfällen, Ausfällen mit einem Nichtlösungsmittel für das Anlagerungssalz oder durch Verdampfen des Lösungsmittels gewonnen.

Die erfindungsgemäßen Ausgangsverbindungen II, die neue und interessante Zwischenprodukte für die Synthese der erfindungsgemäßen Verbindungen I darstellen, werden nach verschiedenen Verfahren hergestellt. So werden sie durch Umsetzung von Pyridazinonen der allgemeinen Formel V

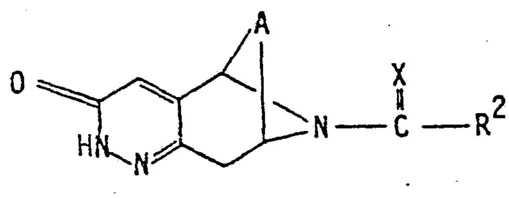 (V),

worin X für ein Sauerstoffatom steht, $R^2$ eine Alkoxygruppe darstellt und A die oben angegebene Bedeutung hat, mit geeigneten Halogenierungsmitteln, wie z.B. mit Phosphoroxidchlorid, Phosphortri- oder -pentachlorid, Phosphoroxidbromid, Oxalylchlorid oder Triphenylphosphin/Tetrachlorkohlenstoff, gegebenenfalls durch anschließenden Austausch des Halogenatoms gegen eine andere Fluchtgruppe Z (beipsielsweise gegen eine Alkoxygruppe, wie z.B. die Methoxygruppe, durch Reaktion mit Natriumalkanolaten, wie z.B. Natriummethanolat; oder gegen eine Alkylmercapto-

gruppe, wie z.B. die Methylmercaptogruppe, durch Reaktion mit Natrium-alkanthiolaten, wie Natriummethanthiolat; oder gegen eine Alkylsulfinyl- oder Alkylsulfonylgruppe, wie z.B. die Methylsulfinyl oder die Methylsulfonylgruppe, durch Einführung einer Alkylmercaptogruppe, wie einer Methylmercaptogruppe und deren anschließende Oxidation, beispielsweise mit äquivalenten Mengen Wasserstoffperoxid) erhalten. Die Umsetzung erfolgt beispielsweise in inerten Lösungsmitteln, z.B. Kohlenwasserstoffen, wie Benzol oder Toluol, bei Temperaturen bis zu 150°C [analog H.E.Baumgarten, P.L.Creger und C.H.Villars, J.Amer.Chem.Soc. 80, 6609 (1958)].

Die Ausgangsverbindungen der Formel II werden ferner durch Umsetzung von Pyridazinen der Formel VI

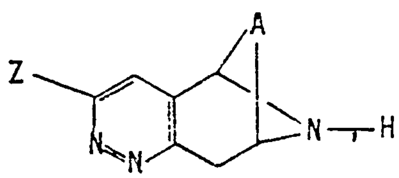

(VI),

worin Z und A die oben angegebene Bedeutung haben, mit Verbindungen des Typs VII

$$R^2—C(=X)Y \qquad \text{(VII)},$$

worin $R^2$ und X die oben angegebene Bedeutung haben und Y eine Fluchtgruppe, vorzugsweise ein Halogenatom darstellt, erhalten. Die Reaktion wird zweckmäßigerweise in einem inerten Lösungsmittel, z.B. einem chlorierten Kohlenwasserstoff, wie Chloroform oder Ethylenchlorid, in Gegenwart eines säurebindenden Mittels, z.B. eines Alkalimetallcarbonates, wie Natrium- oder Kaliumcarbonat, oder eines tertiären Amins, z.B. Triethylamin oder Pyridin, durchgeführt.

Wenn $R^2$ die Bedeutung $-N(R^6)R^7$ hat, wobei $R^6$ ein Wasserstoffatom und $R^7$ einen Alkyl- oder Arylrest bedeutet, so können die Vorprodukte II auch durch Umsetzung eines Heterokumulens VIII

$$R^7—N=C=X \qquad \text{(VIII)},$$

wobei $R^7$ einen Alkyl- oder Arylrest darstellt und X die oben angegebene Bedeutung hat, mit den Pyridazinen VI erhalten werden.

Die Reaktion wird zweckmäßigerweise in einem inerten Lösungsmittel, z.B. einem Kohlenwasserstoff, wie Benzol, Cyclohexan, oder in einem Ether, wie z.B. Diethylether, oder in einem chlorierten Kohlenwasserstoff, wie z.B. Methylenchlorid, durchgeführt.

Die erfindungsgemäßen Ausgangsverbindungen II', die neue und interessante Zwischenprodukte für die Synthese der erfindungsgemäßen Pyridazine I darstellen, erhält man, indem man Pyridazine der Formel VI

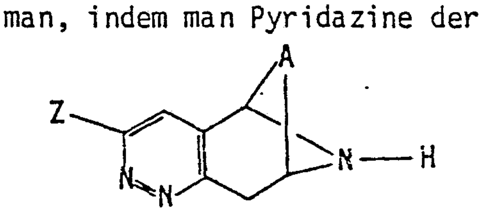 (VI),

worin Z und A die oben angegebene Bedeutung haben, mit Hydrazinderivaten III

$$H_2N - R^1 \quad (III),$$

worin $R^1$ die oben angegebene Bedeutung hat, umsetzt und gegebenenfalls anschließend in die Säureadditionssalze überführt, oder erhaltene Reaktionsprodukte der Formel II', in denen $R^1$ eine $-NHR^3$-Gruppe und $R^3$ ein Wasserstoffatom darstellt, derivatisiert oder substituiert und/oder in die Säureadditionssalze überführt. Die Reaktion wird wie weiter oben beschrieben (Umsetzung von II mit III) durchgeführt.

Verbindungen der Formel V, in denen X ein Sauerstoffatom ist, $R^2$ eine Alkoxygruppe darstellt und A die oben angegebene Bedeutung hat, können aus Dihydropyridazinonen der allgemeinen Formel IX

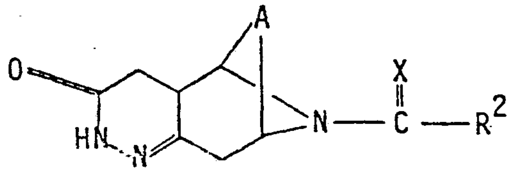 (IX),

worin X, $R^2$ und A die vorstehend angegebene Bedeutung haben, nach dem Fachmann bekannten Dehydrierungsverfahren hergestellt werden.

Als Dehydrierungsmittel kommen Chloranil, 2,3-Dichlor-5,6-dicyan-p-benzochinon, N-Bromsuccinimid und vorzugsweise Brom in Frage. Als Lösungsmittel finden hauptsächlich halogenierte Kohlenwasserstoffe Verwendung, beispielsweise Chloroform oder Ethylenchlorid. Die bevorzugte Reaktionstemperatur ist der Siedepunkt des jeweiligen Lösungsmittels [analog H.E.Baumgarten, P.L.Creger und C.H.Villars, J.Amer.Chem.Soc. 80, 6609 (1958)].

Die erfindungsgemäßen Pyridazine VI werden beispielsweise aus den Pyridazinonen X

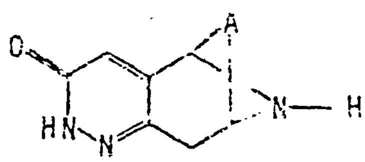

(X),

worin A die oben angegebene Bedeutung hat, durch Halogenierung und gegebenenfalls anschließende Überführung in das Säureadditionssalz erhalten. Geeignete Halogenierungsmittel sind beispielsweise Phosphoroxidtrichlorid oder -bromid. Die Reaktion erfolgt zweckmäßigerweise in inerten Lösungsmitteln, z.B. Kohlenwasserstoffen, wie Benzol, Toluol, oder ohne Lösungsmittel unter Verwendung des Halogenierungsmittels im Überschuß. Die Reaktionstemperatur beträgt bis zu 120°C.

Verbindungen der Formel IX werden aus Aza-bicycloalkanonen der allgemeinen Formel XI

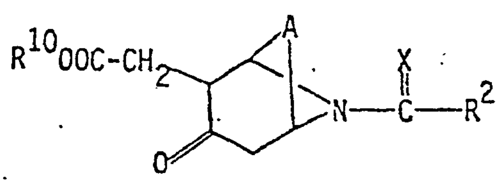

(XI),

wobei X ein Sauerstoffatom ist, $R^2$ eine Alkoxygruppe darstellt, A die oben angegebene Bedeutung hat und $R^{10}$ einen Alkylrest symbolisiert, durch Umsetzung mit Hydrazinhydrat/Eisessig oder einem Hydrazinsalz nach dem Fachmann bekannten Verfahren hergestellt. Die Reaktion erfolgt vorzugsweise

in einem polaren inerten Lösungsmittel, beispielsweise Ethanol. Die Temperatur kann im Bereich zwischen 70 und 110°C, insbesondere bei der Siedetemperatur des Reaktionsgemisches liegen.

Verbindungen der Formel X werden beispielsweise aus Pyridazinonen V hergestellt, wobei X ein Sauerstoffatom ist, $R^2$ eine Alkoxygruppe darstellt und A die oben angegebene Bedeutung hat. Die Abspaltung der $-\overset{X}{\overset{\|}{C}}-R^2$ -Gruppe erfolgt nach dem Fachmann bekannten Verfahren, beispielsweise durch Erhitzen von V in saurem Medium, z.B. in Salzsäure, oder alkalisch, z.B. durch Erhitzen mit einem Alkalimetallhydroxid, wie Natrium- oder Kaliumhydroxid, in einem höhersiedenden Alkohol, wie z.B. n-Butanol.

Verbindungen der Formel XI können nach dem Fachmann bekannten Verfahren aus den Enaminen XII

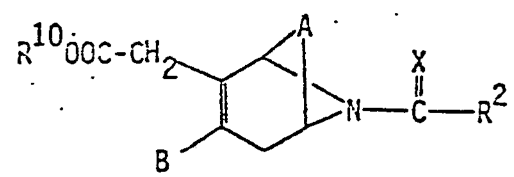 (XII),

worin X ein Sauerstoffatom ist, $R^2$ eine Alkoxygruppe darstellt, A und $R^{10}$ die oben angegebenen Bedeutungen haben und B eine sekundäre, vorzugsweise cyclische Aminogruppe, z.B. eine Pyrrolidino-, Morpholino- oder Piperidinogruppe darstellt, durch Hydrolyse hergestellt werden. Die Hydrolyse erfolgt durch Erhitzen mit Wasser, gegebenenfalls unter Zugabe von verdünnter Lauge, wie z.B. Natronlauge oder Ammoniaklösung, oder unter Zusatz von verdünnter Säure, wie z.B. Salzsäure.

Zu Verbindung IX gelangt man auch in einem dem Fachmann bekannten Verfahren, indem man XII mit Hydrazinhydrat/Eisessig oder mit einem Hydrazinsalz in einem polaren, aprotischen Lösungsmittel, beispielsweise Acetonitril, bei Temperaturen zwischen 70 und 110°C, vorzugsweise bei der Siedetemperatur des Reaktionsgemisches, zur Reaktion bringt.

Verbindung XII erhält man nach dem Fachmann bekannten Verfahren, indem man das Enamin XIII

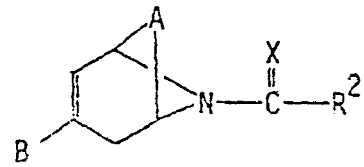

(XIII),

worin X ein Sauerstoffatom ist, $R^2$ eine Alkoxygruppe darstellt und A und B die oben angegebenen Bedeutungen haben, mit Halogenessigsäurealkylester in einem absoluten, bevorzugt polaren Lösungsmittel, wie z.B. Acetonitril oder Ethanol, in Gegenwart eines Protonenacceptors, z.B Kaliumcarbonat oder eines nicht alkylierbaren tertiären organischen Amins, wie z.B. Diisopropylethylamin oder Dicyclohexylethylamin, zur Reaktion bringt [vgl. G.Stork, R.Terrell und J.Szmuszkovicz, J.Amer.Chem.Soc. 76, 2029 (1954)].

Verbindung XIII erhält man nach dem Fachmann bekannten Verfahren, indem man ein Aza-bicyclo-alkanon der allgemeinen Formel XIV

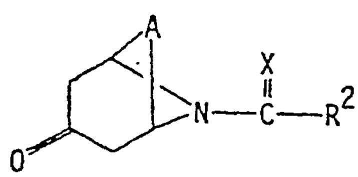

(XIV),

worin X ein Sauerstoffatom ist, $R^2$ eine Alkoxygruppe darstellt und A die oben angegebene Bedeutung hat, mit einem entsprechenden sekundären Amin, wie z.B. Pyrrolidin, Morpholin oder Piperidin, in Gegenwart einer stark wasserentziehenden Lewissäure, vorzugsweise Titantetrachlorid in einem inerten Kohlenwasserstoff, wie z.B. Hexan oder Petrolether, bei 0 bis 50°C zur Reaktion bringt [vgl. W.H.White und H.Weingarten, J.Org.Chem. 32, 213 (1967)].

Zu Verbindungen der Formel XIV gelangt man nach dem Fachmann bekannten Verfahren durch Umsetzung eines Aza-bicyclo-alkanons der allgemeinen Formel XV

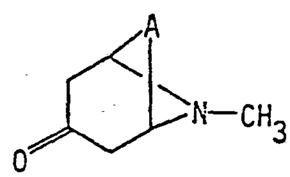

(XV),

worin A die oben angegebene Bedeutung hat, mit einer Verbindung der allgemeinen Formel

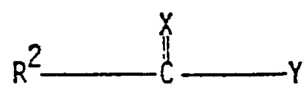

worin X ein Sauerstoffatom ist, $R^2$ eine Alkoxygruppe darstellt und Y eine Fluchtgruppe, z.B. ein Halogenatom, darstellt. Die Reaktion erfolgt vorzugsweise durch Erhitzen in einem inerten Lösungsmittel, z.B. einem Kohlenwasserstoff, wie etwa Benzol oder Toluol, oder in einem Halogenkohlenwasserstoff, wie z.B. Chloroform, 1,2-Dichlorethan oder Trichlorethylen, gegebenenfalls unter Inertgasatmosphäre, auf Temperaturen zwischen 80 bis 120°C, vorzugsweise auf Siedetemperatur des Reaktionsgemisches. Die Reaktionsdauer kann 10 bis 36 Stunden betragen [vgl. Th.A.Montzka, J.D.Matiskella und R.A.Partyka, Tetrahedron Letters 1974, 1325-1327].

Die Verbindungen der Formel XV können nach Vorschriften von A.C.Cope, H.L.Dryden Jr. und Ch.F.Howell [Org.Synth., Coll.Vol.IV, 816] bzw. C.Schöpf und G.Lehmann [Ann. 518, 1 (1935)], hergestellt werden.

Die folgenden Beispiele erläutern die Erfindung näher, ohne sie einzuschränken. Die Abkürzung F. bzw. Sdp. bedeutet Schmelzpunkt bzw. Siedepunkt; Z. bedeutet Zersetzung.

## Beispiel 1

3-Hydrazino-5,7-propano-5,6,7,8-tetrahydro-6-pyrido-[4,3-c]- -pyridazincarbonsäureethylester

$R^1 = -NH_2$, $R^2 = -OC_2H_5$, $A = -(CH_2)_3-$, $X = O$

Eine Suspension von 17,5 g 3-Chlor-5,7-propano-5,6,7,8-tetrahydro-6-pyrido-[4,3-c]-pyridazincarbonsäureethylester in 150 ml Hydrazinhydrat wird bei einer Ölbadtemperatur von 110°C unter Stickstoffatmosphäre und Rühren 1 Stunde unter Rückfluß zum Sieden erhitzt, wobei nach ca. 10 Minuten vollständige Auflösung eintritt, Die gelbe Lösung wird im Vakuum eingeengt und der Rückstand in Chloroform aufgenommen. Die erhaltene Lösung wird zweimal mit Wasser gewaschen und über Natriumsulfat getrocknet, filtriert und im Vakuum zur Trockne eingedampft. Der zurückbleibende hellgelbe, erstarrte Schaum wird in 80 ml Methanol aufgenommen. Nach Zugabe von 7,21 g Fumarsäure und kurzem Rühren wird eine klare, hellgelbe Lösung erhalten, aus der innerhalb 12 Stunden bei 0°C die Titelverbindung als Fumarat auskristallisiert. Nach Absaugen, Waschen mit wenig eiskaltem Methanol und Acetonitril und Trocknen bei 40°C erhält man 19 g (79 %) des Fumarates vom Schmelzpunkt 148 - 150°C (Z.)

Die Ausgangsverbindung kann wie folgt hergestellt werden:

a) 3-Chlor-5,7-propano-5,6,7,8-tetrahydro-6-pyrido-[4,3-c]- -pyridazincarbonsäureethylester:

22,75 g 5,7-Propano-2,3,5,6,7,8-hexahydro-3-oxo-6-pyrido-[4,3-c]- -pyridazincarbonsäureethylester werden in 100 ml Phosphoroxidtrichlorid aufgeschlämmt und 1 Stunden unter Rückfluß zum Sieden erhitzt. Nach dem Abkühlen wird das überschüssige Phosphoroxidtrichlorid im Vakuum abdestilliert und der braune Rückstand auf 150 g Eis gegossen und gerührt. Die saure wässrige Lösung wird

filtriert und dann dreimal mit Methylenchlorid extrahiert. Die vereinigten organischen Phasen werden mit Wasser gewaschen, wobei durch Zugabe von gesättigter Natriumhydrogencarbonatlösung in der wässrigen Phase ein pH von 8 eingestellt wird, über Natriumsulfat getrocknet und dann im Vakuum zur Trockne eingeengt. Der Rückstand wird in einem Gemisch Methylenchlorid/Methanol 9 : 1 aufgenommen, über Kieselgel filtriert und mit Methylenchlorid/Methanol 9 : 1 solange eluiert, bis dünnschichtchromatographisch im ablaufenden Filtrat kein Produkt mehr nachgewiesen werden kann. Das Filtrat wird im Vakuum eingeengt und der Rückstand aus 25 ml Tetrachlorkohlenstoff und 5 ml n-Hexan umkristallisiert.
Ausbeute 18,5 g (75,5 %) F.: 113 - 113,5$^{o}$C.

b) 5,7-Propano-2,3,5,6,7,8- hexahydro -3-oxo-6-pyrido-[4,3-c]-pyridazincarbonsäureethylester:

113 g 5,7-Propano-2,3,4,4a,5,6,7,8-octahydro-3-oxo-6-pyrido-[4,3-c]-pyridazincarbonsäureethylester werden in 490 ml Chloroform gelöst und unter Rückfluß zum Sieden erhitzt. In die siedende Lösung werden innerhalb von 1,5 Stunden unter Rühren 68,1 g Brom, gelöst in 130 ml Chloroform, zugetropft. Nach weiteren 2,5 Stunden Rühren unter Rückfluß läßt man abkühlen und versetzt mit 190 ml Wasser. Unter Rühren wird die wässrige Phase mit gesättigter Natriumhydrogencarbonatlösung auf pH 7 eingestellt und danach die Chloroformphase abgetrennt. Die wässrige Phase wird noch zweimal mit Chloroform extrahiert; die vereinigten Chloroformphasen werden über Magnesiumsulfat getrocknet. Nach dem Abfiltrieren des Trockenmittels wird das Chloroform im Vakuum abgezogen und der Rückstand (116,7 g) ohne weitere Reinigung weiterverarbeitet.

c) 5,7-Propano-2,3,4,4a,5,6,7,8-octahydro-3-oxo-6-pyrido-[4,3-c]-pyridazincarbonsäureethylester:

260 g 3-(1-Pyrrolidinyl)-9-azabicyclo-[3.3.1]-non-2-en-9-carbonsäureethylester und 127,1 g N,N-Diisopropylethylamin werden in 750 ml absolutem Acetonitril gelöst. Zu dieser Lösung wird unter Stickstoffatmosphäre und Rühren die Lösung von 164,2 g Bromessigsäureethylester in 770 ml absolutem Acetonitril bei 25 bis 30°C innerhalb von 2,5 Stunden zugetropft. Die Mischung wird innerhalb einer Stunde langsam erwärmt und dann 4,5 Stunden unter Rückfluß zum Sieden erhitzt. Nach dem Abkühlen werden 54,2 g Hydrazinhydrat und danach 65 g Eisessig zugegeben. Nach weiteren 2,5 Stunden unter Rückfluß und anschließendem Abkühlen wird das Lösungsmittel im Vakuum abgedampft. Nach dem Auflösen des Rückstandes in Chloroform wird zweimal mit Wasser, einmal mit gesättigter Natriumhydrogencarbonatlösung und wieder zweimal mit Wasser gewaschen und über Natriumsulfat getrocknet. Nach dem Abfiltrieren des Trockenmittels wird die Lösung zur Trockne eingedampft. Der Rückstand wird in 250 ml heißem Tetrachlorkohlenstoff gelöst und in der Hitze mit 175 ml n-Hexan langsam versetzt. Nach mehrtägigem Stehen bei 0°C wird das auskristallisierte Produkt abgesaugt und mit wenig kaltem Tetrachlorkohlenstoff gewaschen. Nach dem Trocknen (65°C, im Vakuum) erhält man 113 g (43,3 %) vom F. 123 - 124°C.

d) 3-(1-Pyrrolidinyl)-9-azabicyclo-[3.3.1]-non-2-en-9-carbonsäureethylester:

Zu einer Lösung von 223 g 9-Azabicyclo-[3.3.1]-nonan-3-on-9-carbonsäureethylester und 300 g Pyrrolidin in 2,5 l absolutem Petrolether (Sdp. 50 - 70°C) wird unter Stickstoffatmosphäre und Rühren eine Lösung von 110 g Titantetrachlorid in 450 ml Petrolether innerhalb von 2,5 Stunden bei Raumtemperatur zugetropft. Die Mischung wird 24 Stunden gerührt. Der entstandene hellgelbe Niederschlag wird filtriert, in Benzol aufgeschlämmt, abgesaugt und mit Benzol nachgewaschen. Die vereinigten Filtrate werden im Vakuum eingeengt; das zurückbleibende orange-rote Öl wird durch Vakuumdestillation gereinigt. Man erhält 260 g (93,5 %) gelbes, viskoses Öl, Sdp.: 140°C bei 6,7 Pa.

e) 9-Azabicyclo-[3.3.1]-nonan-3-on-9-carbonsäureethylester:

186,8 g Pseudopelletierin und 264 g frisch destillierter Chlorameisensäureethylester in 1 l absolutem Toluol werden innerhalb einer Stunde langsam zum Sieden erhitzt. Es setzt eine heftige Gasentwicklung ein. Der Ansatz wird weitere 16 Stunden unter Rückfluß zum Sieden erhitzt, dann abgekühlt und filtriert. Das Filtrat wird im Vakuum eingeengt und der Rückstand durch Vakuumdestillation gereinigt. Man erhält 223 g (86,5 %) hellgelbes Öl, Sdp.: 101°C bei 2,7 Pa.

Beispiel 2

3-Isopropylidenhydrazino-5,7-propano-5,6,7,8-tetrahydro-6-pyrido-[4,3-c]-pyridazincarbonsäureethylester

Eine Suspension von 5,8 g 3-Chlor-5,7-propano-5,6,7,8-tetrahydro-6-pyrido-[4,3-c]-pyridazincarbonsäureethylester (vgl.Beispiel 1a) in 50 ml Hydrazinhydrat wird unter einer Stickstoffatmosphäre 1 Stunde bei einer Ölbadtemperatur von 110°C gerührt. Die so erhaltene gelbe Lösung wird im Vakuum vollständig eingeengt und der Rückstand in Chloroform aufgenommen. Die Chloroformlösung wird zweimal mit Wasser gewaschen, über Natriumsulfat getrocknet, filtriert und im Vakuum zur Trockne eingedampft. Der zurückbleibende hellgelbe, erstarrte Schaum wird in 15 ml Aceton gelöst und die entstandene Lösung 30 Minuten unter Rückfluß zum Sieden erhitzt. Das Lösungsmittel wird zum Großteil im Vakuum abgedampft und der Rückstand mit Diethylether versetzt. Nach kurzer Zeit kristallisiert die Titelverbindung aus. Nach Absaugen und Waschen mit Diethylether erhält man 4,6 g (70 %) vom F. 148-150°C.

Beispiel 3

3-(1-p-Chlorphenylethyliden-hydrazino)-5,7-propano-5,6,7,8-tetrahydro-6-pyrido-[4,3-c]-pyridazincarbonsäureethylester

1,2 g des Fumarats von 3-Hydrazino-5,7-propano-5,6,7,8-tetrahydro-6-pyrido-[4,3-c]-pyridazincarbonsäureethylester (Beispiel 1) und 0,52 g 4-Chloracetophenon werden in 10 ml absolutem Ethanol gelöst und 4 Stunden unter Rückfluß zum Sieden erhitzt. Nach Abdampfen des Lösungsmittels im Vakuum wird der Rückstand aus Methanol umkristallisiert. Man erhält 1,4 g (97,5 %) der Titelverbindung als Semifumarat vom F. 201-202°C.

Beispiel 4

3-Isopropylidenhydrazino-5,7-ethano-5,6,7,8-tetrahydro-6-pyrido-[4,3-c]-pyridazincarbonsäureethylester

Eine Aufschlämmung von 4,8 g 3-Chlor-5,7-ethano-5,6,7,8-tetrahydro-6-pyrido-[4,3-c]-pyridazincarbonsäureethylester in 40 ml Hydrazinhydrat wird eine Stunde lang unter einer Stickstoffatmosphäre bei 110°C gerührt. Die dabei entstandene gelbe Lösung wird im Vakuum zur Trockne eingedampft. Der Rückstand wird in Chloroform aufgenommen, die Chloroformlösung zweimal mit Wasser gewaschen, über Natriumsulfat getrocknet und filtriert. Nach Einengen des Filtrats im Vakuum wird der Rückstand in 20 ml Aceton gelöst und eine Stunde unter Rückfluß zum Sieden erhitzt. Die Lösung wird weitgehend eingeengt und der Rückstand mit Diethylether versetzt. Nach kurzer Zeit kristallisiert die Titelverbindung aus. Man erhält 3,9 g (72 %) vom F. 153-156°C.

Die Ausgangsverbindung kann wie folgt hergestellt werden:

a) 3-Chlor-5,7-ethano-5,6,7,8-tetrahydro-6-pyrido-[4,3-c]-pyridazincarbonsäureethylester:

12 g 5,7-Ethano-2,3,5,6,7,8-hexahydro-3-oxo-6-pyrido-[4,3-c]-pyridazincarbonsäureethylester werden in 100 ml Phosphoroxidtrichlorid gelöst und eine Stunde unter Rückfluß zum Sieden erhitzt. Nach dem Abkühlen wird das überschüssige Phosphoroxidtrichlorid im Vakuum abdestilliert. Der braune Rückstand wird in Chloroform aufgenommen, mit Eiswasser verrührt und die wässrige Phase mit Ammoniaklösung neutralisiert. Die wässrige Phase wird zweimal mit Chloroform extrahiert. Die vereinigten Chloroformphasen werden zweimal mit Wasser gewaschen, über Magnesiumsulfat getrocknet und filtriert. Nach dem Einengen des Filtrats im Vakuum wird der Rückstand aus Tetrachlorkohlenstoff/Petrolether umkristallisiert.. Man erhält 8,94 g (69,5 %) vom F. 84°C (Zers.).

b) 5,7-Ethano-2,3,5,6,7,8-hexahydro-3-oxo-6-pyrido-[4,3-c]-pyridazin-carbonsäureethylester:

Zu der siedenden Lösung von 3,18 g 5,7-Ethano-2,3,4,4a,5,6,7,8-octahydro-3-oxo-6-pyrido-[4,3-c]-pyridazincarbonsäureethylester in 20 ml Chloroform wird die Lösung von 2,02 g Brom in 10 ml Chloroform langsam zugetropft. Die Mischung wird eine Stunde unter Rückfluß zum Sieden erhitzt. Nach dem Abkühlen wird mit Eiswasser versetzt. Die wässrige Phase wird mit Natriumhydrogencarbonat neutralisiert. und mit Chloroform extrahiert. Die vereinigten Chloroformphasen werden über Magnesiumsulfat getrocknet und filtriert. Das nach dem Verdampfen des Lösungsmittels im Vakuum verbleibende Öl wird aus Tetrachlorkohlenstoff/Petrolether umkristallisiert. Ausbeute 2,3 g (73,2 %) vom F. 175-177°C.

c) 5,7-Ethano-2,3,4,4a,5,6,7,8-octahydro-3-oxo-6-pyrido-[4,3-c]-pyridazincarbonsäureethylester:

35,6 g 3-(1-Pyrrolidinyl)-8-azabicyclo[3.2.1]oct-3-en-8-carbonsäureethylester und 18,4 g N,N-Diisopropylethylamin werden unter einer Stickstoffatmosphäre in 100 ml wasserfreiem Acetonitril gelöst. Zu dieser Lösung wird unter kräftigem Rühren die Lösung von 23,7 g Bromessigsäureethylester in 100 ml wasserfreiem Acetonitril innerhalb von 45 Minuten zugetropft. Anschließend wird nach langsamem Erwärmen 1 Stunde unter Rückfluß zum Sieden erhitzt. Das Gemisch wird abgekühlt, nacheinander langsam mit 7,8 g Hydrazinhydrat und 9,4 g Essigsäure versetzt, erneut zum Sieden erhitzt und 1 Stunde bei Siedetemperatur gehalten. Der nach Abdestillieren des Lösungsmittels im Vakuum verbleibende Rückstand wird in 200 ml Chloroform gelöst, zweimal mit je 100 ml Wasser, mit Natriumhydrogencarbonatlösung und wieder mit Wasser gewaschen, über Natriumsulfat getrocknet und filtriert. Das Filtrat wird im Vakuum eingeengt und der Rückstand über Kieselgel chromatographiert (Chloroform/Methanol 99 : 1). Die dünnschichtchromatographisch einheitlichen Fraktionen werden im Vakuum eingedampft

und der Rückstand aus Tetrachlorkohlenstoff/Petrolether umkristallisiert. Man erhält 11,4 g (32 %) vom F. 128-131°C.

d) 3-(1-Pyrrolidinyl)-8-azabicyclo[3.2.1]oct-3-en-8-carbonsäureethylester:
36,6 g 8-Azabicyclo[3.2.1]octan-3-on-8-carbonsäureethylester werden in 500 ml absolutem Petrolether gelöst und mit 52,8 g Pyrrolidin versetzt. Unter kräftigem Rühren wird eine Lösung von 19,4 g Titantetrachlorid in 100 ml Petrolether innerhalb von 70 Minuten zugetropft. Unter leichter Erwärmung der Lösung fällt sofort ein hellgelber Niederschlag aus. Nach 110 Stunden Rühren bei Raumtemperatur läßt man den Niederschlag absitzen und dekantiert die gelbe Petroletherlösung. Der Niederschlag wird in 250 ml wasserfreiem Benzol aufgeschlämmt, abgesaugt und mit Benzol gewaschen. Die vereinigten organischen Phasen werden im Vakuum eingeengt; das zurückbleibende Öl wird durch Vakuumdestillation gereinigt. Man erhält 42,8 g (92,2 %) hellgelbes, viskoses Öl. Sdp.: 125-128°C bei 8 Pa.

e) 8-Azabicyclo[3.2.1]octan-3-on-8-carbonsäureethylester:
56,5 g Tropinon werden in 330 ml wasserfreiem Benzol gelöst und unter einer Stickstoffatmosphäre auf 70°C erhitzt. Eine Lösung von 88 g frisch destilliertem Chlorameisensäureethylester in 200 ml Benzol wird innerhalb einer Stunde unter Rühren zugetropft. Nach weiteren 4 Stunden Erhitzen unter Rückfluß wird abgekühlt, filtriert, das Filtrat im Vakuum eingeengt und der Rückstand durch Vakuumdestillation gereinigt. Man erhält 70,75 g (88,4 %) farbloses Öl.
Sdp.: 91-93°C bei 12 Pa.

Beispiel 5

6-Benzoyl-3-hydrazino-5,7-propano-5,6,7,8-tetrahydro-pyrido-[4,3-c]-pyridazin

8,6 g 6-Benzoyl-3-chlor-5,7-propano-5,6,7,8-tetrahydro-pyrido[4,3-c]-pyridazin werden unter einer Stickstoffatmosphäre in einem Gemisch aus 28 ml Dioxan und 28 ml Hydrazinhydrat aufgeschlämmt und zum Sieden

erhitzt. Die Mischung wird 9 Stunden bei Siedetemperatur gehalten, abgekühlt und im Vakuum zur Trockne eingeengt. Der Rückstand wird zwischen Dichlormethan und Wasser verteilt, die organische Phase dreimal mit Wasser gewaschen, über Magnesiumsulfat getrocknet und filtriert. Das Filtrat wird im Vakuum eingeengt und bis zur Gewichtskonstanz getrocknet; man erhält 8,6 g Rohbase als erstarrter Schaum. Die Rohbase wird in 8 ml Methanol gelöst und unter Rühren die Suspension von 3,1 g Fumarsäure in 36 ml Methanol zugegeben. Innerhalb kurzer Zeit entsteht eine klare Lösung aus der nach Zugabe von Ethylacetat, Anreiben und Kühlen die Titelverbindung als Fumarat vom F. 157°C (Zersetzung) auskristallisiert.

Die Ausgangsverbindung kann wie folgt hergestellt werden:

a) 6-Benzoyl-3-chlor-5,7-propano-5,6,7,8-tetrahydro-pyrido-[4,3-c]-pyridazin:

Zu 7,3 g 3-Chlor-5,7-propano-5,6,7,8-tetrahydro-pyrido-[4,3-c]-pyridazin in 58 ml 1.2.-Dichlorethan werden unter Eiskühlung und Rühren nacheinander 5,3 g Triethylamin und 5 g Benzoylchlorid, gelöst in 30 ml 1.2-Dichlorethan zugetropft. Nach 2 Stunden Rühren bei Raumtemperatur wird mit 70 ml Wasser versetzt, die wässrige Phase abgetrennt, die organische Phase je einmal mit 1n-Salzsäure-, Natriumhydrogencarbonatlösung und Wasser gewaschen, über Magnesiumsulfat getrocknet und filtriert. Das Filtrat wird im Vakuum eingeengt, der Rückstand in 25 ml Ethanol aufgenommen und angerieben. Das nach Kühlen auskristallisierte Produkt wird abgesaugt, mit Ethanol gewaschen und im Vakuum bei 80°C getrocknet. Man erhält 8,62 g (78,9 %) vom F. 197-198,5°C.

b) 3-Chlor-5,7-propano-5,6,7,8-tetrahydro-pyrido[4,3-c]-pyridazin:

11,08 g 3-Oxo-5,7-propano-2,3,5,6,7,8-hexahydro-pyrido-[4,3-c]-pyridazin-hydrochlorid und 60,5 ml Phosphoroxidtrichlorid werden 7 Stunden unter Rückfluß zum Sieden erhitzt. Das überschüssige Phosphoroxidtrichlorid wird im Vakuum abdestilliert und das zurückbleibende Öl mit Eiswasser versetzt. Die wässrige Lösung wird mit konzentrierter Ammoniaklösung auf pH 11 eingestellt und viermal mit Chloroform extrahiert. Die vereinigten Chloroformlösungen werden

über Magnesiumsulfat getrocknet, filtriert, mit Aktivkohle bei Raumtemperatur gerührt und wieder filtriert. Das nach dem Einengen des Filtrats kristallisierende Produkt wird im Vakuum getrocknet und ohne weitere Reinigung weiter umgesetzt. Ausbeute: 9,46 g (92,8 %) F.: 146-148°C.

c) 3-Oxo-5,7-propano-2,3,5,6,7,8-hexahydro-pyrido-[4,3-c]-pyridazin-hydrochlorid:
14,5 g 3-Oxo-5,7-propano-2,3,5,6,7,8-hexahydro-6-pyrido-[4,3-c]-pyridazincarbonsäureethylester (vgl. Beispiel 1b) werden in 77 ml konzentrierter Salzsäure gelöst und 30 Stunden unter Rückfluß zum Sieden erhitzt. Die erkaltete Lösung wird einmal mit Chloroform extrahiert und die wässrige Phase im Vakuum zur Trockne eingedampft. Der Rückstand wird mit 50 ml Methanol versetzt, angerieben, die Aufschlämmung zum Sieden erhitzt, gekühlt und abgesaugt. Nach dem Trocknen im Vakuum erhält man 11,2 g (89 %) vom F. 360-362°C (Zers.).

Beispiel 6

6-Benzoyl-3-isopropylidenhydrazino-5,7-propano-5,6,7,8-tetrahydro-pyrido-[4,3-c]-pyridazin

Ein Gemisch aus 4,85 g 6-Benzoyl-3-chlor-5,7-propano-5,6,7,8-tetrahydro-pyrido-[4,3-c]-pyridazin (vgl. Beispiel 5a), 16 ml Hydrazinhydrat und 16 ml Dioxan wird 14 Stunden unter Rückfluß zum Sieden erhitzt. Das Lösungsmittel und das überschüssige Hydrazinhydrat werden im Vakuum abdestilliert. Der Rückstand wird im Hochvakuum getrocknet, in 50 ml Dichlormethan gelöst, die Lösung zweimal mit Wasser gewaschen, über Magnesiumsulfat getrocknet und filtriert. Der nach Einengen des Filtrats im Vakuum verbleibende Rückstand wird in 12 ml Aceton gelöst und 20 Minuten unter Rückfluß zum Sieden erhitzt. Das Lösungsmittel wird im Vakuum abdestilliert und der Rückstand aus Aceton/Diethylether umkristallisiert. Ausbeute: 5,35 g (81,5 %) vom F. 211-212°C (aus Acetonitril).

Beispiel 7

6-Benzoyl-3-(2-butylidenhydrazino)-5,7-propano-5,6,7,8-tetrahydro-pyrido-[4,3-c]-pyridazin

Analog Beispiel 6 erhält man aus 4,95 g 6-Benzoyl-3-chlor-5,7-propano-5,6,7,8-tetrahydro-pyrido-[4,3-c]-pyridazin, 16 ml Hydrazinhydrat, 16 ml Dioxan und 14 ml 2-Butanon 3,5 g (62 %) der Titelverbindung vom F. 180-182°C (aus Acetonitril).

Beispiel 8

3-Isopropylidenhydrazino-6-pivaloyl-5,7-propano-5,6,7,8-tetrahydro-pyrido-[4,3-c]-pyridazin

Ein Gemisch aus 3,84 g 3-Chlor-6-pivaloyl-5,7-propano-5,6,7,8-tetrahydro-pyrido-[4,3-c]-pyridazin, 13 ml Hydrazinhydrat und 13 ml Dioxan wird 12,5 Stunden unter Rückfluß zum Sieden erhitzt. Das Lösungsmittel und überschüssiges Hydrazinhydrat werden im Vakuum abdestilliert. Der Rückstand wird mit Chloroform aufgenommen, die Chloroformlösung zweimal mit Wasser gewaschen, über Magnesiumsulfat getrocknet und filtriert. Das Filtrat wird im Vakuum zur Trockne eingeengt, der Rückstand in 30 ml Aceton gelöst und die Lösung 30 Minuten unter Rückfluß zum Sieden erhitzt. Nach dem Einengen im Vakuum wird der Rückstand aus Acetonitril/Diisopropylether umkristallisiert. Man erhält 2,4 g (55,1 %) vom F. 179-181,5°C.

Die Ausgangsverbindung kann wie folgt hergestellt werden:

3-Chlor-6-pivaloyl-5,7-propano-5,6,7,8-tetrahydro-pyrido-[4,3-c]-pyridazin:

Zu 3 g 3-Chlor-5,7-propano-5,6,7,8-tetrahydro-pyrido-[4,3-c]-pyridazin (vgl. Beispiel 5b) in 25 ml 1.2-Dichlorethan werden unter Eiskühlung und Rühren nacheinander 2,17 g Triethylamin und 1,9 g Pivaloylchlorid (gelöst in 25 ml 1.2-Dichlorethan) zugetropft. Die Lösung wird über Nacht stehengelassen, zweimal mit Wasser, mit 2-n-Salzsäurelösung, wieder mit Wasser und mit Natriumhydrogencarbonatlösung gewaschen, über Natriumsulfat getrocknet und

im Vakuum zur Trockne eingeengt. Der Rückstand (3,84 g, 91,4 %) wird ohne Reinigung weiterverarbeitet.

Beispiel 9

3-Isopropylidenhydrazino-5,7-propano-5,6,7,8-tetrahydro-6-pyrido-[4,3-c]-pyridazincarbonsäure-N-methylamid

4,25 g 3-Chlor-5,7-propano-5,6,7,8-tetrahydro-6-pyrido-[4,3-c]-pyridazincarbonsäure-N-methylamid, 16 ml Hydrazinhydrat und 16 ml Dioxan werden 6 Stunden unter Rückfluß zum Sieden erhitzt. Überschüssiges Hydrazinhydrat und das Lösungsmittel werden im Vakuum abdestilliert, der Rückstand im Hochvakuum getrocknet und in heißem Aceton gelöst. Beim Abkühlen kristallisiert die Titelverbindung als Hydrochlorid aus und wird nochmals aus Aceton/Methanol umkristallisiert. Ausbeute: 3,68 g (68,4 %) F.: 200-201°C (Zers.).

Die Ausgangsverbindung kann wie folgt hergestellt werden:

3-Chlor-5,7-propano-5,6,7,8-tetrahydro-6-pyrido-[4,3-c]-pyridazincarbonsäure-N-methylamid:

Eine Lösung von 1,5 g Methylisocyanat in 30 ml 1.2-Dichlorethan wird innerhalb von 30 Minuten unter Rühren zu einer eisgekühlten Lösung von 5 g 3-Chlor-5,7-propano-5,6,7,8-tetrahydro-pyrido-[4,3-c]-pyridazin (vgl. Beispiel 5b) in 60 ml 1.2-Dichlorethan zugetropft. Nach weiteren 30 Minuten Rühren bei Raumtemperatur wird das Lösungsmittel im Vakuum abdestilliert und der zurückbleibende Schaum in Methanol gelöst. Nach Anreiben und Kühlen erhält man 5,3 g (83,3 %) vom F. 162-165°C.

Beispiel 10

3-(2-Ethoxycarbonylhydrazino)-5,7-propano-5,6,7,8-tetrahydro-6-pyrido-[4,3-c]-pyridazincarbonsäureethylester

4,5 g 3-Chlor-5,7-propano-5,6,7,8-tetrahydro-6-pyrido-[4,3-c]- pyridazincarbonsäureethylester (vgl. Beispiel 1a) und 3,35 g Hydrazincarbonsäure-

ethylester in 30 ml Dioxan werden 8,5 Stunden unter Rückfluß zum Sieden erhitzt. Es wird im Vakuum zur Trockne eingedampft. Der Rückstand in Chloroform gelöst, die Chloroformlösung zweimal mit Wasser gewaschen, über Natriumsulfat getrocknet und filtriert. Das Filtrat wird im Vakuum zur Trockne eingedampft und der zurückbleibende Schaum in siedendem Ethanol gelöst. Beim Abkühlen kristallisiert die Titelverbindung aus. Man erhält 2,17 g vom F. 180,5-182,5°C. Aus der Mutterlauge werden nach Eindampfen und Kristallisation aus Wasser/Methanol weitere 1,95 g vom F. 180,5-183,5°C erhalten. Gesamtausbeute: 4,12 g (78,8 %).

Beispiel 11

3-Hydrazino-5,7-propano-5,6,7,8-tetrahydro-6-pyrido-[4,3-c]-pyridazin-carbonsäureethylester

Zu einer konzentrierten wässrigen Lösung von 0,5 g 3-Hydrazino-5,7-propano-5,6,7,8-tetrahydro-6-pyrido-[4,3-c]-pyridazincarbonsäureethylester-fumarat (vgl. Beispiel 1) werden 0,513 g Embonsäure (1,1'-Methylen-bis-2-hydroxy-3-naphthoesäure) in 2,54 ml 1n-Kaliumhydroxidlösung gegeben. Das Embonat der Titelverbindung fällt augenblicklich aus, wird nach ca. 30 Minuten abgesaugt, gründlich mit Wasser gewaschen und bei 40°C im Vakuum getrocknet. Man erhält 0,8 g (93 %) vom F. 179°C (Zersetzung).

Beispiel 12

3-Isopropylidenhydrazino-6-(N-phenyl-thiocarbamoyl)-5,7-propano-5,6,7,8-tetrahydro-pyrido[4,3-c]pyridazin

2,5 g (10,2 mmol) 3-Isopropylidenhydrazino-5,7-propano-5,6,7,8-tetrahydro-pyrido[4,3-c]pyridazin werden in 25 ml Dichlormethan suspendiert. Unter Rühren und Eiskühlung werden 1,38 g (10,2 mmol) Phenylisothiocyanat zugegeben. Die entstandene gelbe Lösung wird noch 45 Minuten gerührt und dann am Rotationsverdampfer eingeengt. Der Rückstand wird aus Ethanol/Petrolether umkristallisiert. Man erhält 3,5 g (90 %) der Titelverbindung vom F. 133°C (Z.).

BAD ORIGINAL

Beispiel 13

3-Isopropylidenhydrazino-6-(N-phenylcarbamoyl)-5,7-propano-5,6,7,8-tetra-hydro-pyrido[4,3-c]pyridazin

Analog Beispiel 12 erhält man aus 2 g (8,16 mmol) 3-Isopropylidenhydrazino-5,7-propano-5,6,7,8-tetrahydro-pyrido[4,3-c]pyridazin und 0,98 g (8,16 mmol) Phenylisocyanat 2,73 g (92 %) der Titelverbindung vom F. 232°C (Z.).

Beispiel 14

3-Isopropylidenhydrazino-4,7-propano-5,6,7,8-tetrahydro-pyrido[4,3-c]-pyridazin

20 g (63 mmol) 3-Isopropylidenhydrazino-5,7-propano-5,6,7,8-tetrahydro-6-pyrido[4,3-c]pyridazincarbonsäureethylester werden mit 120 ml konzentrierter Salzsäure 72 Stunden unter Rückfluß zum Sieden erhitzt. Nach dem Abkühlen wird mit 200 ml Wasser verdünnt und mit 6 n Natronlauge ein pH von 8 bis 9 eingestellt. Die wäßrige Phase wird zweimal mit Dichlormethan extrahiert, mit 6 n Natronlauge auf pH 10 bis 11 eingestellt, erneut einmal mit Dichlorethan extrahiert und mit konzentrierter Salzsäure auf pH 1 eingestellt. Der nach dem Einengen zurückbleibende Rückstand wird mit Aceton, Dichlormethan und festem Kaliumcarbonat versetzt. Die entstandene Lösung wird abfiltriert, eingeengt und der Rückstand aus Ethanol umkristallisiert. Ausbeute 5,81 g vom F. 219-222°C.

Beispiel 15

3-Isopropylidenhydrazino-5,7-propano-5,6,7,8-tetrahydro-6-pyrido[4,3-c]-pyridazincarbonsäureethylester

Zu 1,0 g 3-Isopropylidenhydrazino-5,7-propano-5,6,7,8-tetrahydropyrido-[4,3-c]pyridazin, aufgeschlämmt in 20 ml wasserfreiem Dichlormethan, werden 0,35 g Triethylamin gegeben. Unter Eiskühlung und Rühren werden

0,34 g Chlorameisensäureethylester zugetropft. Die erhaltene orange Lösung wird eine weitere Stunde bei Raumtemperatur gerührt, zweimal mit 10 ml Wasser und mit 3 ml 0,1n Salzsäurelösung extrahiert, über Natriumsulfat getrocknet und im Vakuum zur Trockne eingeengt. Der zurückbleibende Schaum wird in heißem Aceton gelöst. Nach dem Abkühlen wird die Lösung mit Diethylether versetzt. Die Titelverbindung kristallisiert in der Kälte aus. Man erhält 0,4 g vom F. 148 - 150°C.

Beispiel 16

3-Isopropylidenhydrazino-5,7-propano-5,6,7,8-tetrahydro-pyrido[4.3-c]-pyridazin

2,1 g 3-Chlor-5,7-propano-5,6,7,8-tetrahydropyrido[4,3-c]pyridazin werden unter einer Stickstoffathmosphäre in 25 ml Hydrazinhydrat aufgeschlämmt und 1,5 Stunden unter Rückfluß zum Sieden erhitzt. Nach dem Abkühlen wird filtriert und das Filtrat im Vakuum zur Trockne eingeengt. Der verbleibende Rückstand wird in 30 ml Aceton gelöst, 30 Minuten zum Sieden erhitzt und wieder eingeengt. Das zurückbleibende Öl wird in 50 ml Dichlormethan/Aceton (1:1) gelöst und mit verdünnter Kaliumcarbonat-Lösung ausgeschüttelt. Die wäßrige Phase wird mit Dichlormethan extrahiert. Die vereinigten organischen Phasen werden über Kaliumcarbonat getrocknet, filtriert und eingeengt. Der Rückstand wird aus Ethanol kristallisiert. Man erhält 1,63 g (66,5 %) der Titelverbindung, F. 219 - 221°C.

Beispiel 17

Tabletten mit einem Wirkstoffgehalt von 10 mg

5,0 kg 3-Hydrazino-5,7-propano-5,6,7,8-tetrahydro-6-pyrido[4,3-c]-pyridazincarbonsäureethylester-fumarat, 50,0 kg Calciumsulfatdihydrat, 31,0 kg Maisstärke und 3,0 kg Polyvinylpyrrolidon werden mit 20 Liter Wasser befeuchtet und durch ein Sieb mit 1,25 mm Maschenweite granuliert.

BAD ORIGINAL

Das Granulat wird im Wirbelschichttrockner bis zu einer relativen Feuchte von 50 - 60 % getrocknet und dann mit 8,0 kg Natriumcarboxymethylcellulose, 2,0 kg Talkum und 1,0 kg Magnesiumstearat versetzt. Das fertige Granulat wird zu Tabletten à 200 mg und 8 mm Durchmesser verpreßt.

Beispiel 18

Ampullen mit einem Wirkstoffgehalt von 22,5 mg

1,125 kg 3-Hydrazino-5,7-propano-5,6,7,8-tetrahydro-6-pyrido[4,3-c]-pyridazincarbonsäureethylester-fumarat werden in 80 Liter aqua bidestillata gelöst und mit 4,337 kg Mannit versetzt. Die Lösung wird mit aqua bidestillata auf 100 kg aufgefüllt, über einen Filter sterilfiltriert und in 2-ml-Ampullen abgefüllt.

Beispiel 19

10.000 Kapseln mit einem Wirkstoffgehalt von 5 mg

50 g 3-Hydrazino-5,7-propano-5,6,7,8-tetrahydro-6-pyrido[4,3-c]-pyridazincarbonsäureethylester-fumarat, 945 g mikrokristalline Cellulose und 5 g amorphe Kieselsäure (der Wirkstoff in feingepulverter Form) werden gut vermischt und in Hartgelatinekapseln Größe 4 abgefüllt.

Beispiel 20

250.00 Retard-Kapseln mit einem Wirkstoffgehalt von 30 mg

8,25 kg 3-Hydrazino-5,7-propano-5,6,7,8-tetrahydro-6-pyrido[4,3-c]-pyridazincarbonsäureethylester-fumarat und 3,1 kg Hydroxypropylmethylcellulose werden in einem Gemisch von 16 l 96 %igem Ethanol und 24 l Wasser gelöst. In diese Lösung wird 1,1 kg Talkum suspendiert. In einem Wirbelschichtgerät werden 16 kg Zuckerpellets mit dieser Wirkstofflösung besprüht. Die erhaltenen Wirkstoffpellets werden im gleichen Gerät mit einer Lösung aus 0,6 kg Ethylcellulose, 0,3 kg Hydroxypropylmethylcellulose und 0,12 kg Stearinsäure in 14,8 l Methylenchlorid und 4,0 l 96 %igem Ethanol besprüht. Nach dem Trocknen der überzogenen Wirkstoffpellets werden diese in Hartgelatinekapseln der Größe 4 abgefüllt.

Beispiel 21

10.000 Suppositorien mit einem Wirkstoffgehalt von 20 mg

200 g 3-Hydrazino-5,7-propano-5,6,7,8-tetrahydro-6-pyrido[4,3-c]-pyridazincarbonsäureethylester-fumarat werden in einer Mühle auf eine Korngröße von unter 20 µm vermahlen. 20 kg Neutralfett werden geschmolzen und der Wirkstoff wird in diese Schmelze mit einem hochtourigen Rührwerk homogen eingearbeitet. Die flüssige Suppositorienmasse wird im Vakuum entgast und in Zäpfchenformen gegossen.

## Pharmakologie

Die erfindungsgemäßen substituierten Pyridazine besitzen wertvolle pharmakologische Eigenschaften, insbesondere senken sie den Blutdruck, wie aus Untersuchungen an wachen, genetisch hypertonen Ratten hervorgeht, wobei sie sich bekannten Verbindungen, wie z.B. 3-Hydrazino-5,6,7,8-tetrahydro-6-pyrido-[4,3-c]-pyridazincarbonsäureethylester, 3-Hydrazino-4-methyl-5,6,7,8-tetrahydro-6-pyrido-[4,3-c]-pyridazincarbonsäureethylester-hydrochlorid und Hydralazin überlegen erweisen.

Zur Bestimmung der antihypertensiven Wirkung werden die Verbindungen bzw. Gemische in den angegebenen Dosen an zwei aufeinanderfolgenden Tagen an je 8 Ratten (Stamm Kyoto) mit genetisch bedingtem Hochdruck täglich einmal mittels Schlundsonde verabfolgt. Die Messung des Blutdrucks erfolgte jeweils 6 und 24 Stunden nach Substanzgabe.

Die Messung des systolischen Blutdrucks erfolgt mit Hilfe einer um die Schwanzwurzel gelegten, aufblasbaren Manschette, an der distal ringförmig 3 piezoelektrische Kristalle (im Abstand von 120°) zur Registrierung der Pulswellen angebracht sind. Die Bestimmung des Blutdrucks geschieht durch Aufblasen der Manschette und graphische Aufzeichnung der Pulsamplitude [analog H.Friebel und E.Vreden, Arch.exper.Path.u. Pharmakol. 232, 419 (1958)]. Die Aufbewahrung der Tiere und die Durchführung der Versuche erfolgte bei 22 - 24°C und 50 - 60 % Luftfeuchtigkeit. Zur Gewöhnung an den Messvorgang wurde an den Tieren drei Tage lang jeweils 2 bis 3 mal täglich eine Messung vorgenommen. Hierfür, wie in den nachfolgenden Versuchen, wurden die Ratten in tunnelförmige Drahtkäfige eingebracht; eine Schmalseite der Käfige ist verschiebbar, die andere weist eine Öffnung zur Durchführung des Schwanzes auf. Um eine bessere Durchblutung der Schwanzarterie während der Messung zu gewährleisten, werden die Schwänze 5 - 10 Minuten mit einer Rotlichtlampe (150 Watt) bestrahlt (Abstand: Tier - Lampe = 50 cm). Die Lufttemperatur unmittelbar am Schwanz beträgt 30 - 33°C. Der Körper der Tiere wird abgedeckt und so vor direkter Bestrahlung geschützt.

Die Toxizitätsuntersuchungen werden an weiblichen NMRI-Mäusen (Körpergewicht 22 - 26 g) durchgeführt. Die Tiere (5 Tiere pro Dosis) erhalten Futter und Wasser ad libitum. Verschiedene Dosen der Substanzen werden oral verabreicht. Die Beobachtungsdauer beträgt 14 Tage. Die $LD_{50}$, d.h. die Dosis bei der 50 % der Tiere sterben, wird aus der Dosiswirkungskurve graphisch ermittelt.

In den anschließenden Tabellen werden die untersuchten Verbindungen durch eine laufende Nummer gekennzeichnet, die wie folgt zugeordnet sind:

| lfd. Nr. | Name der Verbindung |
|---|---|
| 1 | 3-Hydrazino-5,6,7,8-tetrahydro-6-pyrido-[4,3-c]-pyridazin-carbonsäureethylester |
| 2 | 3-Hydrazino-4-methyl-5,6,7,8-tetrahydro-6-pyrido-[4,3-c]--pyridazincarbonsäureethylester-hydrochlorid |
| 3 | Hydralazin |
| 4 | 3-Hydrazino-5,7-propano-5,6,7,8-tetrahydro-6-pyrido-[4,3-c]-pyridazincarbonsäureethylester-fumarat |
| 5 | 6-Benzoyl-3-isopropylidenhydrazino-5,7-propano-5,6,7,8-tetra-hydro-pyrido-[4,3-c]-pyridazin |
| 6 | 3-Isopropylidenhydrazino-5,7-ethano-5,6,7,8-tetrahydro-6-pyrido-[4,3-c]-pyridazincarbonsäureethylester |

Tabelle I gibt für die Vertreter der erfindungsgemäßen Verbindungen die prozentuale Senkung des Blutdrucks nach oraler Verabreichung bei der Ratte und die letale Wirkung nach oraler Gabe bei der Maus wieder.

Zur Ereichung einer mindestens äquieffektiven Blutdrucksenkung benötigt man z.B. fünf- bis zehnfach höhere Dosen (µMol/kg) von Hydralazin als von Verbindungen der Beispiele 4 bis 6. Über die

überlegene Wirkungsdauer der erfindungsgemäßen Verbindungen informieren die $24^h$-Werte, die im Gegensatz zu den Vergleichsverbindungen besonders am zweiten Tag eine deutlich gesteigerte Blutdrucksenkung aufzeigen, obwohl die Untersuchungen zum Teil mit der Hälfte der ursprünglichen Dosis fortgeführt wurden. Außerdem ist zu berücksichtigen, daß die erfindungsgemäßen Verbindungen drei- bis viermal weniger toxisch sind als Hydralazin.

Tabelle I

%-Änderung des Blutdrucks (BP) an genetisch hypertonen Ratten nach täglich einmaliger p.o.-Applikation an zwei aufeinanderfolgenden Tagen (N = 8/Dosis) und Toxizität (p.o.;$LD_{50}$) an Mäusen (N=5/Dosis)

| lfd. Nr. | Dosis (mg/kg) | Dosis (µMol/kg) | BP (%-Änderung) 1.Tag 6 h | BP (%-Änderung) 1.Tag 24 h | BP (%-Änderung) 2.Tag 6 h | BP (%-Änderung) 2.Tag 24 h | $LD_{50}$ (mg/kg) |
|---|---|---|---|---|---|---|---|
| 1 | 10 | 42,1 | -24,5 | -6,0 | - | - | 340 |
| 1 | 5 | 21,05 | - | - | -17,5 | 8,5 | |
| 2 | 20 | 79,6 | - 4,7 | -1,0 | - 4,7 | -3,7 | |
| 3 | 20 | 125,0 | -10,7 | -1,5 | -11,8 | -6,1 | 122* |
| 4 | 2,5 | 9,0 | - 9,0 | -11,8 | -15,9 | -17,1 | 400 |
| 5 | 10 | 28,6 | -20,1 | -4,4 | - | - | 510 |
| 5 | 5 | 14,3 | - | - | -15,2 | -12,8 | |
| 6 | 10 | 33,0 | -23,1 | -20,5 | - | - | 300 |
| 6 | 5 | 16,5 | - | - | -21,5 | -23,1 | |

* L.Dorigotti, R.Rolandi, C.Carpi, Pharmacol. Res. Commun. 8, 295 - 398 (1976)
E.Baldoni, A.Sardi, V.Dezulian, M.Capellini, G.Bianchi, Arzneim. Forschung (Drug Research) 23, 1591 - 95 (1973)

Die Tabelle II gibt für die Vertreter der erfindungsgemäßen Verbindungen die prozentuale Änderung des Blutdrucks (BP) genetisch hypertoner Ratten ($24^h$-Wert des 2. Tages) an und verdeutlicht die Überlegenheit der erfindungsgemäßen Verbindungen über die bekannten Vergleichsverbindungen anhand der berechneten therapeutischen Wirksamkeit (Th.W.)

Tabelle II

Therapeutische Wirksamkeit (Th.W.) errechnet aus der Relation zwischen prozentualer Änderung des Blutdrucks (BP) genetisch hypertoner Ratten nach 2 Tagen ($24^h$-Wert des 2. Tages) und der applizierten Dosis (µMol/kg)

| lfd. Nr. | BP (%-Änderung) 2. Tag, 24 h | Dosis (µMol/kg) | Th.W. [%-Änderung BP / µMol/kg] | Th. W. [lfd.Nr. 3=1,0] |
|---|---|---|---|---|
| 1 | - 8,5 | 21,05 | 0,404 | 8,3 |
| 2 | - 3,7 | 79,6 | 0,046 | 0,9 |
| 3 | - 6,1 | 125,0 | 0,0488 | 1,0 |
| 4 | - 17,1 | 9,0 | 1,90 | 38,9 |
| 5 | - 12,8 | 14,3 | 0,87 | 17,8 |
| 6 | - 23,1 | 16,5 | 1,40 | 28,7 |

In Tabelle III ist die Erniedrigung des Blutdrucks genetisch hypertoner Ratten bei oraler Applikation von 3-Hydrazino-5,7-propano-5,6,7,8-tetrahydro-6-pyrido-[4,3-c]-pyridazin-carbonsäureethylester-fumarat dargestellt. Jeweils 8 Tiere erhielten an 10 aufeinanderfolgenden Tagen einmal täglich die angegebene Dosis.

Tabelle III

Erniedrigung des Blutdruckes genetisch hypertoner Ratten (in mm Hg), bei Gabe verschiedener Dosen von Nr. 4 (in mg/kg Ratte), gemessen an 10 aufeinanderfolgenden Tagen jeweils 6 Stunden nach Applikation

| | Erniedrigung des Blutdrucks (in mm Hg) | | | |
|---|---|---|---|---|
| Tag | a | b | c | d |
| 1 | 25,0 | 35,0 | 48,0 | 58,0 |
| 2 | 14,0 | 59,0 | 67,0 | 77,5 |
| 3 | 20,0 | 44,0 | 53,0 | 66,0 |
| 4 | 21,0 | 67,0 | 72,0 | 74,0 |
| 5* | - | - | - | - |
| 6* | - | - | - | - |
| 7 | 27,0 | 53,0 | 59,0 | 69,0 |
| 8 | 21,0 | 66,0 | 67,0 | 66,0 |
| 9 | - | 64,0 | 63,0 | 61,0 |
| 10 | - | 71,0 | 66,0 | 63,0 |

*) Samstag/Sonntag keine Messungen

a = Dosis 1,25 mg/kg

b = Dosis 2,5 mg/kg

c = Dosis 5,0 mg/kg

d = Dosis 10,0 mg/kg

Patentansprüche (BE, CH, DE, FR, GB, IT, LU, NL, SE)

1. Substituierte Pyridazine der allgemeinen Formel H

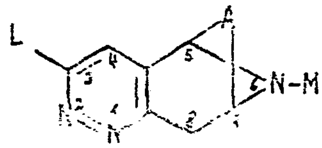 (H),

worin

A eine Niederalkylengruppe,

L eine Fluchtgruppe Z oder die Gruppe $-NH-R^1$,

M ein Wasserstoffatom oder die Gruppe $-C(=X)R^2$,

$R^1$ eine gegebenenfalls substituierte oder derivatisierte Aminogruppe,

$R^2$ eine Alkylgruppe, eine Alkoxygruppe, eine Alkylmercaptogruppe, eine gegebenenfalls substituierte Arylgruppe, eine Phenalkoxygruppe oder eine gegebenenfalls substituierte Aminogruppe und

X ein Sauerstoffatom oder ein Schwefelatom darstellt,

sowie ihre Säureadditionssalze.

2. Substituierte Pyridazine nach Anspruch 1, gekennzeichnet durch die allgemeine Formel (I)

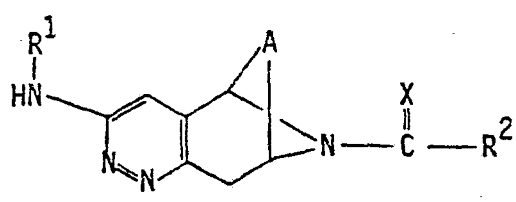 (I),

worin

A eine Niederalkylengruppe,

$R^1$ eine gegebenenfalls substituierte oder derivatisierte Aminogruppe,

$R^2$ eine Alkylgruppe, eine Alkoxygruppe, eine Alkylmercaptogruppe, eine gegebenenfalls substituierte Arylgruppe, eine Phenalkoxygruppe oder eine gegebenenfalls substituierte Aminogruppe darstellt und

X ein Sauerstoffatom oder ein Schwefelatom bedeutet,

sowie ihre Säureadditionssalze.

3. Substituierte Pyridazine nach Anspruch 1, gekennzeichnet durch die allgemeine Formel (I*)

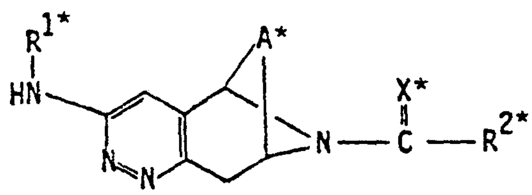

(I*),

worin

A* einen Alkylenrest mit 2 bis 3 Kohlenstoffatomen,

$R^{1*}$ eine -NH-$R^{3*}$-Gruppe oder eine -N=C($R^{8*}$)$R^{9*}$-Gruppe,

$R^{2*}$ eine Niederalkylgruppe, eine Niederalkoxygruppe, eine gegebenenfalls substituierte Phenylgruppe oder eine -N($R^{6*}$)$R^{7*}$-Gruppe,

$R^{3*}$ ein Wasserstoffatom, eine Niederalkanoylgruppe oder eine Niederalkoxycarbonylgruppe,

$R^{6*}$ ein Wasserstoffatom oder eine Niederalkylgruppe,

$R^{7*}$ eine Niederalkylgruppe oder eine gegebenenfalls substituierte Phenylgruppe bedeuten oder

$R^{6*}$ und $R^{7*}$ gemeinsam unter Einschluß des Stickstoffatoms einen Piperidino-, Morpholino-, Piperazino- oder 4-Niederalkylpiperazinorest darstellen,

$R^{8*}$ eine Niederalkylgruppe oder eine gegebenenfalls substituierte Phenylgruppe,

$R^{9*}$ eine Niederalkylgruppe oder eine gegebenenfalls substituierte Phenylgruppe und

X* ein Sauerstoffatom bedeutet,

sowie ihre Säureadditionssalze.

4. Substituierte Pyridazine nach Anspruch 1, gekennzeichnet durch die allgemeine Formel I**

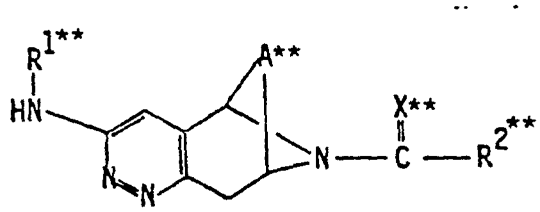

(I**),

worin

A** einen Alkylenrest mit 2 bis 3 Kohlenstoffatomen,

$R^{1**}$ eine $-NH-R^{3**}$-Gruppe oder eine $-N=C(R^{8**})R^{9**}$-Gruppe,

$R^{2**}$ eine Niederalkyl- oder Niederalkoxygruppe, eine Phenylgruppe oder eine $-N(R^{6**})R^{7**}$-Gruppe,

$R^{3**}$ ein Wasserstoffatom oder eine Niederalkoxycarbonylgruppe,

$R^{6**}$ ein Wasserstoffatom,

$R^{7**}$ eine Niederalkylgruppe oder eine Phenylgruppe,

$R^{8**}$ eine Niederalkylgruppe,

$R^{9**}$ eine Niederalkylgruppe oder eine gegebenenfalls halogen-monosubstituierte Phenylgruppe und

X** ein Sauerstoffatom bedeutet,

sowie ihre Säureadditionssalze.

5. Substituierte Pyridazine nach Anspruch 1, gekennzeichnet durch die allgemeine Formel II

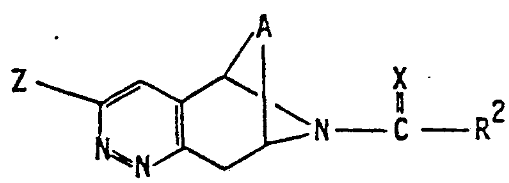

(II),

worin

A eine Niederalkylengruppe,

Z eine Fluchtgruppe,

$R^2$ eine Alkylgruppe, eine Alkoxygruppe, eine Alkylmercaptogruppe, eine gegebenenfalls substituierte Arylgruppe, eine Phenalkoxygruppe oder eine gegebenenfalls substituierte Aminogruppe darstellt und

X ein Sauerstoffatom oder ein Schwefelatom bedeutet.

6. Substituierte Pyridazine nach Anspruch 1, gekennzeichnet durch die allgemeine Formel II'

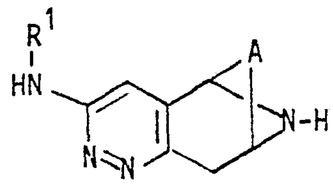

(II'),

worin

A eine Niederalkylengruppe und

$R^1$ eine gegebenenfalls substituierte oder derivatisierte Aminogruppe darstellt,

sowie ihre Säureadditionssalze.

7. Substituierte Pyridazine nach Anspruch 1, gekennzeichnet durch die allgemeine Formel VI

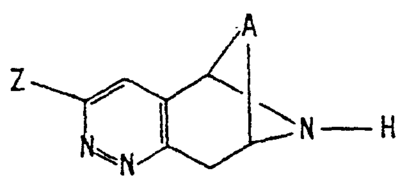

(VI),

worin

A eine Niederalkylengruppe und

Z eine Fluchtgruppe darstellt,

sowie ihre Säureadditionssalze.

8. Arzneimittel, enthaltend eine oder mehrere Verbindungen nach Anspruch 2 oder 3 oder 4 und/oder ihre pharmakologisch verträglichen Säureadditionssalze mit anorganischen und organischen Säuren.

9. Verfahren zur Herstellung von substituierten Pyridazinen der allgemeinen Formel I

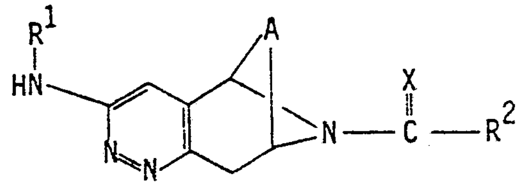 (I),

worin

A eine Niederalkylengruppe,

$R^1$ eine gegebenenfalls substituierte oder derivatisierte Aminogruppe,

$R^2$ eine Alkylgruppe, eine Alkoxygruppe, eine Alkylmercaptogruppe, eine gegebenenfalls substituierte Arylgruppe, eine Phenalkoxygruppe oder eine gegebenenfalls substituierte Aminogruppe und

X ein Sauerstoffatom oder ein Schwefelatom darstellt,

sowie ihren Säureadditionssalzen,

dadurch gekennzeichnet, daß man ein Pyridazin der allgemeinen Formel II

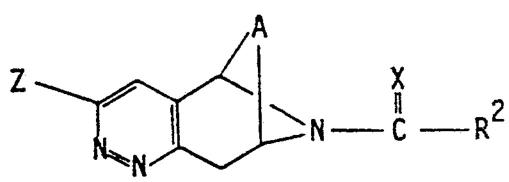 (II),

worin A, $R^2$ und X die vorstehend angegebene Bedeutung haben und Z eine Fluchtgruppe darstellt, mit einem Hydrazinderivat der allgemeinen Formel III

$$H_2N \text{———} R^1 \qquad \text{(III)},$$

worin $R^1$ die oben angegebene Bedeutung hat, umsetzt und gegebenenfalls anschließend in die Säureadditionssalze überführt oder erhaltene Reaktionsprodukte der Formel I, in denen $R^1$ eine $-NHR^3$-Gruppe und $R^3$ ein Wasserstoffatom darstellt, derivatisiert oder substituiert und/oder in die Säureadditionssalze überführt; oder daß man ein Pyridazin

der allgemeinen Formel II'

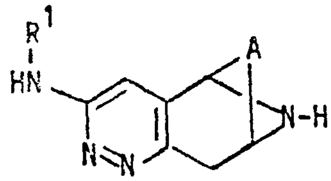

(II'),

worin $R^1$ und A die vorstehend angegebene Bedeutung haben, mit einer Verbindung der allgemeinen Formel VII

$$R^2\text{—}C(=X)Y \qquad (VII),$$

worin $R^2$ und X die oben angegebene Bedeutung haben und Y eine Fluchtgruppe darstellt, oder (wenn $R^2$ die Bedeutung $-NHR^7$ hat und $R^7$ einen Alkyl- oder Arylrest darstellt) mit einem Heterokumulen VIII

$$R^7\text{—}N{=}C{=}X \qquad (VIII),$$

worin $R^7$ einen Alkyl- oder Arylrest und X ein Sauerstoff- oder Schwefelatom darstellt, umsetzt und gegebenenfalls anschließend in die Säureadditionssalze überführt, oder erhaltene Reaktionsprodukte der Formel I, in denen $R^1$ eine $-NHR^3$-Gruppe und $R^3$ ein Wasserstoffatom darstellt, derivatisiert oder substituiert und/oder in die Säureadditionssalze überführt.

10. Verfahren nach Anspruch 9, dadurch gekennzeichnet, daß man Verbindungen der Formel II mit Verbindungen der Formel III umsetzt und gegebenenfalls anschließend in die Säureadditionssalze überführt oder erhaltene Reaktionsprodukte der Formel I, in denen $R^1$ eine $-NHR^3$-Gruppe und $R^3$ ein Wasserstoffatom darstellt, derivatisiert oder substituiert und/oder in die Säureadditionssalze überführt.

Patentansprüche

1. Verfahren zur Herstellung von substituierten [illegible] der allgemeinen Formel I

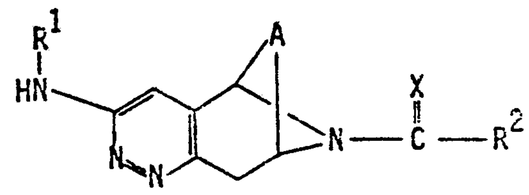

(I),

worin

A eine Niederalkylengruppe,

$R^1$ eine gegebenenfalls substituierte oder derivatisierte Aminogruppe,

$R^2$ eine Alkylgruppe, eine Alkoxygruppe, eine Alkylmercaptogruppe, eine gegebenenfalls substituierte Arylgruppe, eine Phenalkoxygruppe oder eine gegebenenfalls substituierte Aminogruppe und

X ein Sauerstoffatom oder ein Schwefelatom darstellt,

sowie ihren Säureadditionssalzen,

dadurch gekennzeichnet, daß man ein Pyridazin der allgemeinen Formel II

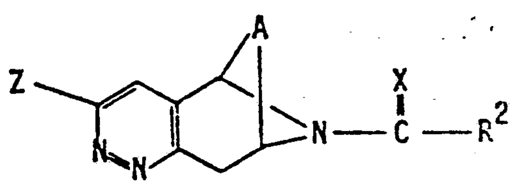

(II),

worin A, $R^2$ und X die vorstehend angegebene Bedeutung haben und Z eine Fluchtgruppe darstellt, mit einem Hydrazinderivat der allgemeinen Formel III

$$H_2N \text{———} R^1 \qquad \text{(III)},$$

worin $R^1$ die oben angegebene Bedeutung hat, umsetzt und gegebenenfalls anschließend in die Säureadditionssalze überführt oder erhaltene Reaktionsprodukte der Formel I, in denen $R^1$ eine $-NHR^3$-Gruppe und $R^3$ ein Wasserstoffatom darstellt, derivatisiert oder substituiert und/oder in die Säureadditionssalze überführt; oder daß man ein Pyridazin

der allgemeinen Formel II'

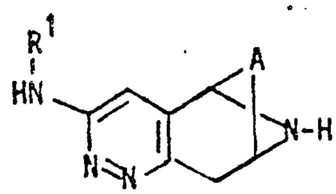

(II'),

worin $R^1$ und A die vorstehend angegebene Bedeutung haben, mit einer Verbindung der allgemeinen Formel VII

$$R^2—C(=X)Y \qquad (VII),$$

worin $R^2$ und X die oben angegebene Bedeutung haben und Y eine Fluchtgruppe darstellt, oder (wenn $R^2$ die Bedeutung $-NHR^7$ hat und $R^7$ einen Alkyl- oder Arylrest darstellt) mit einem Heterokumulen VIII

$$R^7—N{=}C{=}X \qquad (VIII),$$

worin $R^7$ einen Alkyl- oder Arylrest und X ein Sauerstoff- oder Schwefelatom darstellt, umsetzt und gegebenenfalls anschließend in die Säureadditionssalze überführt, oder erhaltene Reaktionsprodukte der Formel I, in denen $R^1$ eine $-NHR^3$-Gruppe und $R^3$ ein Wasserstoffatom darstellt, derivatisiert oder substituiert und/oder in die Säureadditionssalze überführt.

2. Verfahren nach Anspruch 1 zur Herstellung von substituierten Pyridazinen der allgemeinen Formel I*

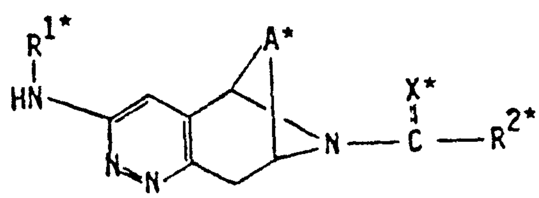

(I*),

worin

$A^*$ einen Alkylenrest mit 2 bis 3 Kohlenstoffatomen,

$R^{1*}$ eine $-NH-R^{3*}$-Gruppe oder eine $-N=C(R^{8*})R^{9*}$-Gruppe,

$R^{2*}$ eine Niederalkylgruppe, eine Niederalkoxygruppe, eine gegebenenfalls substituierte Phenylgruppe oder eine $-N(R^{6*})R^{7*}$-Gruppe,

$R^{3*}$ ein Wasserstoffatom, eine Niederalkanoylgruppe oder eine Niederalkoxycarbonylgruppe,

$R^{6*}$ ein Wasserstoffatom oder eine Niederalkylgruppe,

$R^{7*}$ eine Niederalkylgruppe oder eine gegebenenfalls substituierte Phenylgruppe bedeuten oder

$R^{6*}$ und $R^{7*}$ gemeinsam unter Einschluß des Stickstoffatoms einen Piperidino-, Morpholino-, Piperazino- oder 4-Niederalkylpiperazinorest darstellen,

$R^{8*}$ eine Niederalkylgruppe oder eine gegebenenfalls substituierte Phenylgruppe,

$R^{9*}$ eine Niederalkylgruppe oder eine gegebenenfalls substituierte Phenylgruppe und

$X^*$ ein Sauerstoffatom bedeuten,

sowie ihren Säureadditionssalzen,

dadurch gekennzeichnet, daß man ein Pyridazin der allgemeinen Formel II*

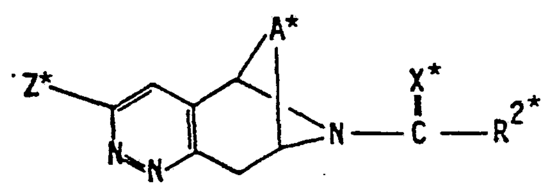

(II*),

worin $A^*$, $R^{2*}$ und $X^*$ die vorstehend angegebene Bedeutung haben und $Z^*$ ein Halogenatom darstellt, mit einem Hydrazinderivat der allgemeinen Formel III*

$$H_2N—R^{1*}$$ (III*),

worin $R^{1*}$ die oben angegebene Bedeutung hat, umsetzt und gegebenenfalls anschließend in die Säureadditionssalze überführt oder erhaltene Reaktionsprodukte der Formel I*, in denen $R^{1*}$ eine $-NHR^{3*}$-Gruppe und $R^{3*}$ ein Wasserstoffatom darstellt, derivatisiert oder substituiert und/oder in die Säureadditionssalze überführt; oder daß man ein Pyridazin der allgemeinen Formel II'*

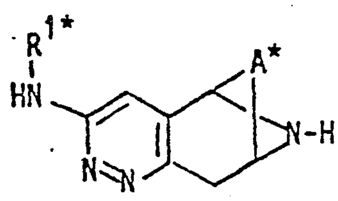

(II'*),

worin $R^{1*}$ und A* die vorstehend angegebene Bedeutung haben, mit einer Verbindung der allgemeinen Formel VII*

$$R^{2*}—C(=X^*)Y^* \qquad (VII^*),$$

worin $R^{2*}$ und X* die oben angegebene Bedeutung haben und Y* ein Halogenatom oder eine Alkoxygruppe darstellt, oder (wenn $R^{2*}$ die Bedeutung $-NHR^{7*}$ hat und $R^{7*}$ die oben angegebene Bedeutung hat) mit einem Heterokumulen VIII*

$$R^{7*}—N{=}C{=}X^* \qquad (VIII^*),$$

worin $R^{7*}$ und X* die oben angegebene Bedeutung haben, umsetzt und gegebenenfalls anschließend in die Säureadditionssalze überführt, oder erhaltene Reaktionsprodukte der Formel I*, in denen $R^{1*}$ eine $-NHR^{3*}$-Gruppe und $R^{3*}$ ein Wasserstoffatom darstellt, derivatisiert oder substituiert und/oder in die Säureadditionssalze überführt.

3. Verfahren nach Anspruch 1 zur Herstellung von substituierten Pyridazinen der allgemeinen Formel I**

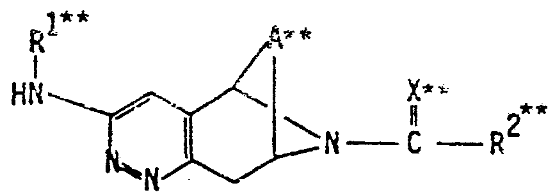

(I**),

worin

A** einen Alkylenrest mit 2 bis 3 Kohlenstoffatomen,

$R^{1**}$ eine $-NH-R^{3**}$-Gruppe oder eine $-N=C(R^{8**})R^{9**}$-Gruppe,

$R^{2**}$ eine Niederalkyl- oder Niederalkoxygruppe, eine Phenylgruppe oder eine $-N(R^{6**})R^{7**}$-Gruppe,

$R^{3**}$ ein Wasserstoffatom oder eine Niederalkoxycarbonylgruppe,

$R^{6**}$ ein Wasserstoffatom,

$R^{7**}$ eine Niederalkylgruppe oder eine Phenylgruppe,

$R^{8**}$ eine Niederalkylgruppe,

$R^{9**}$ eine Niederalkylgruppe oder eine gegebenenfalls halogen-mono-substituierte Phenylgruppe und

X** ein Sauerstoffatom bedeuten,

sowie ihren Säureadditionssalzen,

dadurch gekennzeichnet, daß man ein Pyridazin der allgemeinen Formel II**

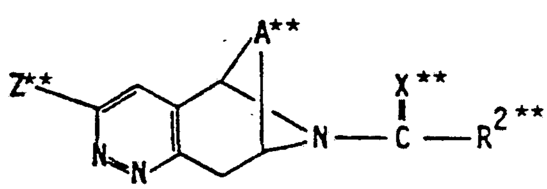

(II**),

worin A**, $R^{2**}$ und X** die vorstehend angegebene Bedeutung haben und Z** ein Brom- oder Chloratom darstellt, mit einem Hydrazinderivat der allgemeinen Formel III**

$$H_2N \text{———} R^{1**}$$ (III**),

worin $R^{1**}$ die oben angegebene Bedeutung hat, umsetzt und gegebenenfalls anschließend in die Säureadditionssalze überführt oder erhaltene

Reaktionsprodukte der Formel I**, in denen $R^{1**}$ eine $-NHR^{3**}$-Gruppe und $R^{3**}$ ein Wasserstoffatom darstellt, derivatisiert oder substituiert und/oder in die Säureadditionssalze überführt; oder daß man ein Pyridazin der allgemeinen Formel II'**

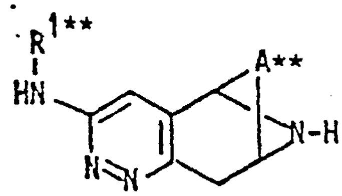

(II'**),

worin $R^{1**}$ und A** die vorstehend angegebene Bedeutung haben, mit einer Verbindung der allgemeinen Formel VII**

$$R^{2**}—C(=X^{**})Y^{**} \qquad (VII^{**}),$$

worin $R^{2**}$ und X** die oben angegebene Bedeutung haben und Y** ein Brom- oder Chloratom oder eine Niederalkoxygruppe darstellt, oder (wenn $R^{2**}$ die Bedeutung $-NHR^{7**}$ hat und $R^{7**}$ die oben angegebene Bedeutung hat) mit einem Heterokumulen VIII**

$$R^{7**}—N{=}C{=}X^{**} \qquad (VIII^{**}),$$

worin $R^{7**}$ und X** die oben angegebene Bedeutung haben, umsetzt und gegebenenfalls anschließend in die Säureadditionssalze überführt, oder erhaltene Reaktionsprodukte der Formel I**, in denen $R^{1**}$ eine $-NHR^{3**}$-Gruppe und $R^{3**}$ ein Wasserstoffatom darstellt, derivatisiert oder substituiert und/oder in die Säureadditionssalze überführt.

4. Verfahren nach Anspruch 1, dadurch gekennzeichnet, daß man Verbindungen der Formel II mit Verbindungen der Formel III umsetzt, und gegebenenfalls anschließend in die Säureadditionssalze überführt oder erhaltene Reaktionsprodukte der Formel I, in denen $R^1$ eine $-NHR^3$-Gruppe und $R^3$ ein Wasserstoffatom darstellt, derivatisiert oder substituiert und/oder in die Säureadditionssalze überführt.

5. Verfahren nach Anspruch 2, dadurch gekennzeichnet, daß man Verbindungen der Formel II* mit Verbindungen der Formel III* umsetzt und gegebenenfalls anschließend in die Säureadditionssalze überführt oder erhaltene Reaktionsprodukte der Formel I*, in denen $R^{1*}$ eine $-NHR^{3*}$-Gruppe und $R^{3**}$ ein Wasserstoffatom darstellt, derivatisiert oder substituiert und/oder in die Säureadditionssalze überführt.

6. Verfahren nach Anspruch 3, dadurch gekennzeichnet, daß man Verbindungen der Formel II** mit Verbindungen der Formel III** umsetzt und gegebenenfalls anschließend in die Säureadditionssalze überführt oder erhaltene Reaktionsprodukte der Formel I**, in denen $R^{1**}$ eine $-NHR^{3**}$-Gruppe und $R^{3**}$ ein Wasserstoffatom darstellt, derivatisiert oder substituiert und/oder in die Säureadditionssalze überführt.

7. Verfahren nach Anspruch 3, dadurch gekennzeichnet, daß man Verbindungen der Formel II**, worin A** einen Alkylenrest mit 2 bis 3 Kohlenstoffatomen, $R^{2**}$ eine tert.-Butyl-, eine Ethoxy-, eine Phenyl- oder eine $-N(R^{6**})R^{7**}$-Gruppe, $R^{6**}$ ein Wasserstoffatom, $R^{7**}$ eine Methylgruppe, X** ein Sauerstoffatom und Z** ein Brom- oder Chloratom darstellt, mit Verbindungen der Formel III**, worin $R^{1**}$ eine $-NH-R^{3**}$- oder eine $-N{=}C(R^{8**})R^{9**}$-Gruppe, $R^{3**}$ ein Wasserstoffatom oder eine Ethoxycarbonylgruppe, $R^{8**}$ eine Methylgruppe und $R^{9**}$ eine Methyl-, Ethyl- oder 4-Chlorphenylgruppe bedeutet, umsetzt und gegebenenfalls anschließend in die Säureadditionssalze überführt, oder erhaltene Reaktionsprodukte der Formel I**, in denen $R^{1**}$ eine $-NHR^{3**}$-Gruppe und $R^{3**}$ ein Wasserstoffatom darstellt, derivatisiert oder substituiert und/oder in die Säureadditionssalze überführt.

8. Verfahren nach Anspruch 3, dadurch gekennzeichnet, daß man Verbindungen der Formel II**, worin A** einen Alkylenrest mit 2 bis 3 Kohlenstoffatomen, $R^{2**}$ eine Ethoxy- oder eine Phenylgruppe, X** ein Sauerstoffatom und Z** ein Brom- oder Chloratom darstellt, mit Verbindungen der Formel III**, worin $R^{1**}$ eine $-NHR^{3**}$- oder eine $-N{=}C(R^{8**})R^{9**}$-Gruppe, $R^{3**}$ ein Wasserstoffatom, $R^{8**}$ eine Methyl-

BAD ORIGINAL

gruppe und $R^{9**}$ eine Methylgruppe bedeutet, umsetzt und gegebenenfalls anschließend in die Säureadditionssalze überführt.

9. Verfahren nach Anspruch 3, dadurch gekennzeichnet, daß man Verbindungen der Formel II**, worin A** einen Alkylenrest mit 2 bis 3 Kohlenstoffatomen, $R^{2**}$ eine Ethoxy- oder eine Phenylgruppe, X** ein Sauerstoffatom und Z** ein Brom- oder Chloratom darstellt, mit Verbindungen der Formel III**, worin $R^{1**}$ eine -$NHR^{3**}$-Gruppe und $R^{3**}$ ein Wasserstoffatom bedeutet, umsetzt und erhaltene Reaktionsprodukte der Formel I** gegebenenfalls derivatisiert oder substituiert und/oder in die Säureadditionssalze überführt.

10. Verfahren nach Anspruch 9, dadurch gekennzeichnet, daß man 3-Hydrazino-5,7-propano-5,6,7,8-tetrahydro-6-pyrido-[4,3-c]-pyridazincarbonsäureethylester und/oder seine Säureadditionssalze herstellt.

0011246

Nummer der Anmeldung

Europäisches Patentamt

# EUROPÄISCHER RECHERCHENBERICHT

EP 79 10 4394

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D | DE - A - 2 221 808 (SANDOZ)<br>* Ansprüche 4,7 *<br>---- | 2,8 |

KLASSIFIKATION DER ANMELDUNG (Int.Cl. 3)

C 07 D 471/18
A 61 K 31/50//
(C 07 D 471/18,
237/00,221/00,
221/00)
(C 07 D 471/18,
237/00,221/00,
209/00)

RECHERCHIERTE SACHGEBIETE (Int. Cl. 3)

C 07 D 471/18
A 61 K 31/50

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 07-02-1980 | ALFARO |